# EUROPEAN PATENT APPLICATION

(11) **EP 4 613 329 A1**
(43) Date of publication of application: **10.09.2025**
(21) Application number: 24161757.0
(22) Date of filing: 06.03.2024
(51) Int. Cl.: A61P 27/06, C07D 405/14, C07D 471/10, A61P 27/02, C07D 405/10

(54) **COMPOUND FOR INHIBITING BRD PROTEIN**

(71) Applicant: Benobio Co., Ltd., Seongnam-si, Gyeonggi-do (KR)
(72) Inventor: LEE, In-hyun, Gwangju (KR); SON, Minhee, Seongnam-si, Gyeonggi-do (KR)
(74) Representative: Isarpatent

(57) **Abstract**

The present disclosure relates to a compound of Chemical Formula I or II, a solvate, a stereoisomer or a pharmaceutically acceptable salt thereof.

## Description

### BACKGROUND

### Field

The present disclosure relates to a novel carboxamide derivative exhibiting BRD protein inhibitory activity and a composition for preventing and treating ophthalmic diseases including the carboxamide derivative as an active ingredient. Particularly, the novel carboxamide derivative of the present disclosure has a BRD inhibitory effect and inhibits retinal degeneration by suppressing inflammatory responses, thereby providing a pharmaceutical composition for preventing and treating various ophthalmic diseases, such as diabetic retinopathy, wet and dry macular degeneration, glaucoma, and uveitis.

### Description of the Related Art

Post-translational modification (PTM) of histone is involved in the regulation of gene expression and chromatin organization in eukaryotic cells. Histone acetylation at a specific lysine residue is PTM regulated by histone acetylase and histone deacetylase. The histone acetylation controls gene expression by mobilizing a protein complex in which a highly conserved protein called a bromodomain binds directly to acetylated lysine in histone and other proteins. There are more than 60 bromodomain-containing proteins in the human genome.

BRD protein includes BRD2, BRD3, BRD4, and BRDT, and except for BRDT, which is localized in the testis, the remaining proteins are widely expressed in various tissues. In addition, it has been reported that the BRD protein family is associated with various diseases, including cancer, metabolic diseases, and inflammation.

For example, oncogenic fusions of BRD4 or BRD3, and nuclear protein in testis (NUT) genes, caused by chromosomal translocations, result in aggressive cancer named NUT midline carcinoma (French et al., J Clin Oncol, 22 (2004), 4135-9; French et al., J Clin Pathol, 63 (2008), 492-6). A BRD3/4 bromodomain is conserved in these fusion proteins, and a knockdown or selective BRD protein inhibitor, JQ1 causes the death of these cancer cells in both *in vitro* and an animal tumor model (Filippakopoulos et al., Nature, 468 (2010), 1067-73). It is known that JQ1 and other selective BRD inhibitors bind to the BRD protein to prevent acetyl-lysine binding, which prevents BRD proteins from interacting with chromatin, thereby preventing transcription from being regulated.

BRD4 was identified as a target in acute myeloid leukemia (AML) by RNAi screen (Zuber et al., Nature, 478 (2011), 524-8). These findings were validated using the BRD inhibitors JQ1 and I-BET151 *in vitro* and *in vivo* (Dawson et al., Nature, 478(2011), 529-33) . It is also known that the BRD inhibitors have broad anticancer activity in acute leukemia, multiple myeloma, and other hematologic malignancies. In several cancer models, acute downregulation of an oncogenic transcription factor Myc has been observed upon BRD inhibition (Delmore et al., Cell, 146 (2011), 904-17; Mertz et al., Proc Natl Acad Sci US A, 108 (2011), 16669-74). Recent studies suggest that the BRD inhibitors have the expandability to be applied to other carcinomas, including lung cancer, brain cancer, and the like.

It has been reported that another BRD inhibitor I-BET762 closely related to JQ1 in its chemical structure and BRD binding mode regulates the expression of key inflammatory genes in a mouse model and protects the human body from endotoxic shock and bacterial-induced sepsis (Nicodeme et al., Nature, 468 (2010), 1119-23). In addition, these results have been used to support clinical evaluation of a BRD inhibitor RVX-208 in clinical trials in patients with atherosclerosis, coronary artery disease, dyslipidemia, diabetes, and other cardiovascular diseases (McNeill, Curr Opin Investig Drugs, 3 (2010), 357-64 and www.clinicaltrials.gov).

It was found that both RVX-208 and I-BET762 upregulated apolipoprotein A-I, which was important in reducing tissue levels of cholesterol. In addition, it is considered that BRD proteins are involved in the regulation of proliferation and transcription of several viruses, so that the BRD inhibitors may have antiviral activity (Weidner-Glunde, Frontiers in Bioscience 15 (2010), 537-549).

Under this background, the present inventors confirmed that a novel BRD-inhibiting low molecular synthetic substance showing excellent inhibitory activity to BRD proteins, an epigenetic recognizer, had an excellent retinal degeneration inhibitory effect capable of preventing and treating ophthalmic diseases caused by retinal degeneration, etc., and then completed the present disclosure.

Although several bromodomain inhibitors are known in clinical and preclinical use, there is an urgent need for the development of new bromodomain inhibitors that can solve the problems of recurrence of diseases and resistance to therapeutic agents and reduce side effects.

Meanwhile, redox reactions exist in many physiological processes, and oxygen molecules are required for life, but may generate reactive molecules that lead to disease. Other reactive chemical species, including free radicals, also cause pathological conditions. It has previously been known that aerobic metabolism related to tissue damage is caused by reactive oxygen species (ROS). The ROS and recently known reactive nitrogen species (RNS), like hormones, are messengers of cell signaling and cause chemical modifications of enzymes and changes in oxidant levels.

In addition, among the 20 essential amino acids, cysteine, methionine, tyrosine, and tryptophan are particularly prone to oxidation. Accordingly, these protein substances that are metabolized in the human body cause various modifications, such as metal binding, disulfide bond formation, methylation, and acetylation.

Currently, research on cellular signal regulation mechanisms has focused on phosphorylation, but the present disclosure was completed by applying redox chemistry technology considering redox control mechanisms such as oxidation, S-nitrosylation, and the like according to a "redox state" for diseases caused by oxidative and nitrosative stress. That is, the present inventors researched and developed a new bromodomain inhibitor in consideration of the redox state, based on the fact that the degree of binding between signaling substances varies depending on the redox state when the BRD inhibitor recognized lysine residues of a histone protein.

The eye consists of the outer membrane, media, inner membrane, refractive medium, etc. The outer membrane consists of the cornea, which is the front surface covering the black pupil, and the sclera behind the cornea, and the media consists of the iris, ciliary body, choroids, etc., and the inner membrane consists of the retina. The lens, vitreous body, and aqueous humor are included in the refractive medium. Functional disorder or loss of the eye is one of factors that greatly deteriorate the quality of life, and it is important to maintain and protect the eye health that deteriorates due to various factors that may adversely affect the eye, such as aging, disease, and vision. Ophthalmic diseases include various diseases, such as retinal diseases including retinal degenerative disease and glaucoma, cataracts, corneal conjunctival epithelial disorders, or corneal epithelial wounds.

Currently, laser therapy, photocoagulation, cryocoagulation, photodynamic therapy, etc. are known as treatments for these ophthalmic diseases. These treatments are all surgery-based treatments, and drug treatments are still in the development stage. Treatment through surgery has a disadvantage of being not applied to all patients, has a low success rate, and is expensive to cause social and economic problems. Unfortunately, most patients who cannot undergo surgery lead to blindness because there is currently no special therapeutic drug. As the human lifespan extends, these ophthalmic diseases continue to increase, so that the development of appropriate therapeutic agents therefor is urgently needed.

Therapeutic agents for ophthalmic diseases currently under development mainly include steroids, matrix metalloproteinase (MMP) inhibitors, angiogenesis inhibitors, antibodies against angiogenic growth factors, etc.

The macula is a nervous tissue located in the center of the retina, and most of visual cells are gathered in the macula, and the macula is an area where images of objects are formed and is mainly responsible for central vision. Macular degeneration is an ophthalmic disease that mostly progresses with age, and degeneration occurs in the macula, causing visual impairment. The macular degeneration is one of the difficult-to-treat ophthalmic diseases that causes blurred vision and distorted near vision in the early stages of the disease, and then causes blindness later.

Age-related macular degeneration is known to be the most common cause of blindness in the elderly, and approximately 30 million people worldwide suffer from the disease, and approximately 500,000 patients lose their vision every year due to the disease. Even in Korea, the age-related macular degeneration is one of three major causes of blindness along with glaucoma and diabetic retinopathy, and its prevalence is gradually increasing with an increase in the elderly population. The age of onset is also decreasing from 60s to middle-aged people in 40s and 50s.

The age-related macular degeneration is broadly classified into two types: exudative (wet) macular degeneration and atrophic (dry) macular degeneration, and the exudative macular degeneration accounts for approximately 10% of age-related macular degeneration and is accompanied by fundus findings, such as choroidal neovascularization, retinal pigment epithelium detachment, sensory retinal detachment, and retinal pigment epithelium rupture, and 70 to 900 of blindness due to age-related macular degeneration is known to be caused by an exudative lesion.

As a recently developed treatment for exudative age-related macular degeneration, intravitreal anti-angiogenic growth factor antibody injection has improved patients' vision and improved the prognosis of the disease. However, the treatment is expensive, has a short half-life of the efficacy, so that it is inconvenient to receive repeated injections every month, and there is a problem of increasing complications such as cataracts, endophthalmitis, vitreous hemorrhage, and retinal detachment due to a surgical method requiring direct drug delivery into the vitreous cavity. Accordingly, there is an urgent need to develop therapeutic agents that solve these side effects occurring during treatment and consider patients' convenience.

Glaucoma is a disease that causes loss of retinal ganglion cells and is closely related to retinal diseases.

Retinal degenerative disease is known as a disease that progresses due to various environmental factors such as genetic or oxidative stress and causes degeneration of photoreceptor cells, resulting in vision loss. In most cases, patients complain of a decrease in peripheral vision, such as night blindness, from the beginning of the disease, and central vision is relatively well preserved, and then vision declines in the later stages. In addition, the glaucoma is a group of diseases consisting of various conditions with various clinical and histopathological findings and shows symptoms such as changes in the optic disc, damage to retinal ganglion cells, and subsequent visual field defects.

The retina is the innermost tissue of the eye belonging to the central nervous system. Retinal degeneration is a pathological phenomenon that is accompanied by retinal diseases such as age-related macular degeneration and retinitis pigmentosa, resulting in the death of photoreceptor cells, which ultimately leads to blindness, and exhibits characteristics of neurodegenerative diseases. Like other neurodegenerative diseases, there is currently no method for inhibiting retinal degeneration, and therefore, retinal disease accompanied by retinal degeneration may be referred to as an incurable disease. Recently, there have been reports that inflammatory response is very important in retinal degeneration, and that retinal degeneration may be inhibited by controlling such neuroinflammation. Many diseases, including neurodegenerative diseases, are accompanied by epigenetic changes, and recent attempts have been made to prevent and treat diseases by controlling the epigenetic changes. As an example, a substance called JQ, known as one of the broad BRD protein inhibitors, showed potential for treating degenerative retinal diseases through microglial cell activation in a retinitis pigmentosa mouse model (Zhao et al, 2017, Photoreceptor protection via blockade of BRD epigenetic readers in a murine model of inherited retinal degeneration. Journal of Neuroinflammation (2017) 14:14, 1-15) .

Under this background, the present inventors confirmed that a novel carboxamide derivative showing excellent inhibitory activity against BRD proteins, an epigenetic recognizer, had an excellent retinal degeneration inhibitory effect capable of preventing and treating ophthalmic diseases caused by retinal degeneration, etc., and then completed the present disclosure.

### SUMMARY

An object to be achieved by the present disclosure is to provide a novel carboxamide derivative with BRD protein inhibitory activity. Another object to be achieved by the present disclosure is to provide a composition for preventing or treating ophthalmic diseases such as diabetic retinopathy, glaucoma, uveitis, wet and dry macular degeneration, age-related macular degeneration, etc. by using an effect of a novel low-molecular compound of the present disclosure to inhibit retinal degeneration, such as reduced inflammation, through epigenetic changes.

An aspect of the present disclosure provides a compound of Chemical Formula I below, a solvate, a stereoisomer or a pharmaceutically acceptable salt thereof:

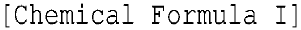

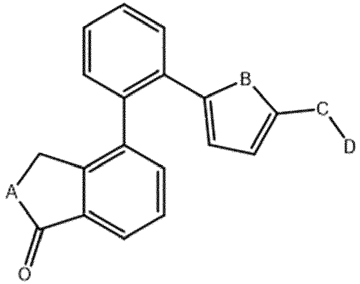

in which,
A and B are each independently any one selected from the group consisting of oxygen, nitrogen, and sulfur, and when A or B is nitrogen, the hydrogen bonded to nitrogen is substituted with an alkyl group,
Cis any one selected from the group consisting of hydrogen, C₁₋₆ alkyl, and carbonyl, and
Dis hydrogen, C₁₋₆ alkyl, -NH₂, -NH(C₁₋₆ alkyl), -N(C₁₋₆ alkyl) (C₁₋₆ alkyl), -NH(C₁₋₆ cycloalkyl), cycloalkyl, heterocycloalkyl, aryl or heteroaryl.

Another aspect of the present disclosure provides a compound of Chemical Formula II below, a solvate, a stereoisomer or a pharmaceutically acceptable salt thereof:

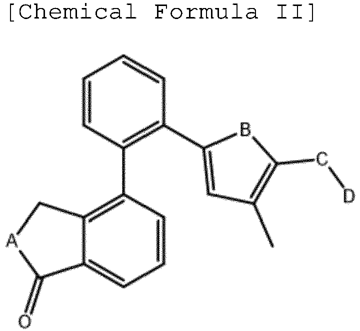

in which,
A and B are each independently any one selected from the group consisting of oxygen, nitrogen, and sulfur, and when A or B is nitrogen, the hydrogen bonded to nitrogen is substituted with an alkyl group,
C is hydrogen or C₁₋₆ alkyl,
D is hydrogen, C₁₋₆ alkyl, -NH₂, -NH(C₁₋₆ alkyl), -N(C₁₋₆ alkyl)(C₁₋₆ alkyl), cycloalkyl, heterocycloalkyl, aryl or heteroaryl,
the cycloalkyl, heterocycloalkyl, aryl, and heteroaryl may be substituted with at least one substituent selected from the group consisting of hydrogen, -OH, =O, -C₁₋₆ alkyl, -C(=O)Ra, -C(=O)N(Ra)(Rb), -C₁₋₆ alkylC(=O)N(Ra) (Rb), and benzyl in which at least one of hydrogen or carbon is substituted with halogen,
the heterocycloalkyl includes at least one selected from the group consisting of pyrrolidinyl, piperidinyl, piperazinyl, methyl piperazinyl, morpholinyl, and piperazinonyl, and
the Ra and Rb are each independently H or C₁₋₆ alkyl.

Yet another aspect of the present disclosure provides a pharmaceutical composition for preventing or treating ophthalmic diseases including the compound, the solvate, the stereoisomer, or the pharmaceutically acceptable salt thereof as an active ingredient.

The ophthalmic disease of the present disclosure includes endophthalmitis, keratitis, conjunctivitis, keratoconjunctivitis, uveitis, blepharitis, scleritis, iritis, glaucoma, retinal degeneration, retinitis pigmentosa, retinal detachment, retinal pigment epithelium detachment, retinal break, diabetic retinopathy, retinopathy of prematurity, polypoidal choroidal vasculopathy, ischemic proliferative retinopathy, cone dystrophy, proliferative vitreoretinopathy, retinal artery occlusion, retinal vein occlusion, Leber's optic atrophy, corneal neovascularization, retina choroidal neovascularization, wet and dry macular degeneration, or age-related macular degeneration, but is not limited thereto.

Preferably, the ophthalmic disease of the present disclosure may be diabetic retinopathy, glaucoma, uveitis, wet and dry macular degeneration, or age-related macular degeneration.

According to the present disclosure, the compound of Chemical Formula I or II, the solvate, the stereoisomer, or the pharmaceutically acceptable salt thereof has an effect of effectively inhibiting retinal degeneration by alleviating the inflammatory responses induced by retinal degeneration through an epigenetic effect of inhibiting BRD proteins. The effect of inhibiting the retinal degeneration can also be used for retinal degeneration and other central nervous diseases by controlling neuroinflammation. Therefore, the compound of Chemical Formula I or II, the solvate, the stereoisomer, or the pharmaceutically acceptable salt thereof can be effectively used to prevent or treat various ophthalmic diseases, such as diabetic retinopathy, glaucoma, uveitis, wet and dry macular degeneration, and age-related macular degeneration.

### BRIEF DESCRIPTION OF THE DRAWINGS

The above and other aspects, features and other advantages of the present disclosure will be more clearly understood from the following detailed description taken in conjunction with the accompanying drawings, in which:
FIG. 1 illustrates results of OCT imaging for Example 21-administered group;
FIG. 2 illustrates results of OCT imaging for an Example 25-administered group;
FIG. 3 illustrates results of OCT imaging for a Comparative Example 4-administered group;
FIG. 4 illustrates results of FFA imaging for an Example 21-administered group;
FIG. 5 illustrates results of FFA imaging for an Example 25-administered group; and
FIG. 6 illustrates results of FFA imaging for a Comparative Example 4-administered group;

### DETAILED DESCRIPTION OF THE EMBODIMENT

Hereinafter, various embodiments of the present disclosure will be described in detail with reference to the accompanying drawings. The present disclosure is not limited to specific embodiments, and it should be understood to include various modifications, equivalents, and/or alternatives to the embodiments of the present disclosure. In connection with the description of the drawings, similar reference numerals may be used for similar components.

In the present specification, expressions such as "have," "may have," "include," or "may include" refer to the presence of the corresponding feature (e.g., numerical value, function, operation, or component such as part), and does not exclude the presence of additional features.

In the present specification, the expression such as "A or B", "at least one of A and/or B", or "one or more of A and/or B" may include all possible combinations of items listed together. For example, "A or B", "at least one of A and B", or "at least one of A or B" may refer to all cases of (1) including at least one A, (2) including at least one B, or (3) including both at least one A and at least one B.

The expression of "configured to" used herein may be changed and used to, for example, "suitable for", "having the capacity to", "designed to", "adapted to", "made to" or "capable of", depending on the situation. The term of "configured to" may not necessarily mean only "specifically designed to".

The terms used herein are used to illustrate only specific embodiments and may not be intended to limit the scope of other embodiments. A singular form may include a plural form unless otherwise clearly meant in the context. The terms used herein, including technical or scientific terms, may have the same meaning as generally understood by those of ordinary skill in the art described in the present disclosure. The terms defined in a general dictionary among the terms used herein may be interpreted in the same or similar meaning as or to the meaning in the context of the related art and will not be interpreted as an ideal or excessively formal meaning unless otherwise defined in the present specification. In some cases, even the terms defined in the present specification cannot be interpreted to exclude the embodiments of the present specification.

The embodiments disclosed in the present specification are presented for explanation and understanding of the disclosed technical contents, and do not limit the scope of the present disclosure. Therefore, the scope of the present specification should be interpreted as including all changes or various other embodiments based on the technical idea of the present disclosure.

Hereinafter, a preferred embodiment of the present disclosure will be described in detail. Terms and words used in the present specification and claims should not be interpreted as being limited to typical or dictionary meanings but should be interpreted as having meanings and concepts which comply with the technical spirit of the present disclosure, based on the principle that an inventor can appropriately define the concept of the term to describe his/her own invention in the best manner.

Therefore, the configurations of the embodiments described in the present specification are merely the most preferred embodiment of the present disclosure and are not intended to represent all of the technical ideas of the present disclosure, and thus, it should be understood that various equivalents and modifications capable of replacing the embodiments at the time of this application.

Throughout the specification, when a part "comprises" a certain component, it is meant that the part may further include another component rather than excluding another component, unless specifically stated to the contrary.

Hereinafter, the present disclosure will be described in detail.

The present disclosure relates to a novel compound represented by Chemical Formula I or II below, and more specifically, to a novel compound having inhibitory activity against BRD proteins and a pharmaceutical composition for preventing or treating BRD protein-related diseases including the novel compound.

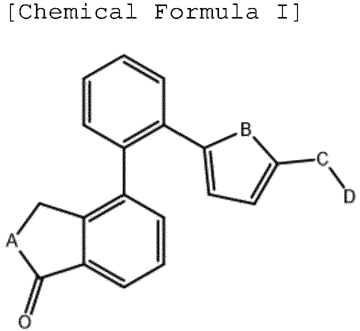

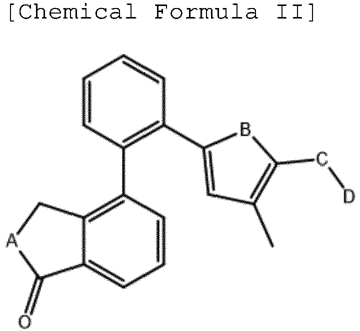

Unless otherwise stated, terms used in the description and claims of the present disclosure have the meanings set forth below.

According to the convention used in the art, in Chemical Formula herein, " 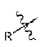 " is used to indicate that a moiety or substituent "R" is attached to a skeleton structure.

"Alkyl" is hydrocarbon having primary, secondary, tertiary and/or quaternary carbon atoms and includes saturated aliphatic groups which may be straight-chain, branched or cyclic, or a combination thereof. For example, the alkyl group may have 1 to 20 carbon atoms (i.e., C₁-C₂₀ alkyl), 1 to 10 carbon atoms (i.e., C₁-C₁₀ alkyl), or 1 to 6 carbon atoms (i.e., C₁-C₆ alkyl). Unless otherwise defined, in a preferred embodiment, the alkyl refers to C₁-C₆ alkyl. Examples of a suitable alkyl group may include methyl (Me, -CH₃), ethyl (Et, -CH₂CH₃), 1-propyl (n-Pr, n-propyl, -CH₂CH₂CH₃), 2-propyl (i-Pr, i-propyl, -CH(CH₃)₂), 1-butyl (n-Bu, n-butyl, -CH₂CH₂CH₂CH₃), 2-methyl-1-propyl (i-Bu, i-butyl, -CH₂CH(CH₃)₂), 2-butyl (s-Bu, s-butyl, -CH(CH₃)CH₂CH₃), 2-methyl-2-propyl (t-Bu, t-butyl, -C(CH₃)₃), 1-pentyl (n-pentyl, - CH₂CH₂CH₂CH₂CH₃), 2-pentyl (-CH (CH₃)CH₂CH₂CH₃), 3-pentyl (-CH(CH₂CH₃)₂), 2-methyl-2-butyl (-C(CH₃)₂CH₂CH₃), 3-methyl-2-butyl (-CH(CH₃)CH(CH₃)₂), 3-methyl-1-butyl (-CH₂CH₂CH(CH₃)₂), 2-methyl-1-butyl (-CH₂CH(CH₃)CH₂CH₃), 1-hexyl (-CH₂CH₂CH₂CH₂CH₂CH₃), 2-hexyl (-CH (CH₃)CH₂CH₂CH₂CH₃), 3-hexyl (-CH(CH₂CH₃)(CH₂CH₂CH₃)), 2-methyl-2-pentyl (-C(CH₃)₂CH₂CH₂CH₃), 3-methyl-2-pentyl (-CH(CH₃)CH(CH₃)CH₂CH₃), 4-methyl-2-pentyl (-CH(CH₃)CH₂CH(CH₃)₂), 3-methyl-3-pentyl (-C(CH₃)(CH₂CH₃)₂), 2-methyl-3-pentyl (-CH(CH₂CH₃)CH(CH₃)₂), 2,3-dimethyl-2-butyl (-C(CH₃)₂CH(CH₃)₂), 3,3-dimethyl-2-butyl (-CH (CH₃)C(CH₃)₃), and octyl (-(CH₂)₇CH₃), but are not limited thereto.

Moreover, the term "alkyl" as used throughout the specification, Examples and claims is intended to include both unsubstituted and substituted alkyl groups, the latter thereof refers to an alkyl residue having a substituent that replaces hydrogen on one or more carbons of the hydrocarbon backbone, including a haloalkyl group such as trifluoromethyl and 2,2,2-trifluoromethyl, and the like.

When used with a chemical moiety such as acyl, acyloxy, alkyl, alkenyl, alkynyl or alkoxy, it is considered that the term "C_{x-y}" or "Cₓ-C_{y}" includes groups containing x to y carbons in the chain. C₀ alkyl represents hydrogen when the group is located at a terminal position, and a bond when the group is located inside. For example, a (C₁-C₆)alkyl group contains 1 to 6 carbon atoms in the chain.

"Alkoxy" refers to a group with Chemical Formula -O-alkyl in which the alkyl group as defined above is attached to a parent compound through oxygen atoms. The alkyl moiety of the alkoxy group may have, for example, 1 to 20 carbon atoms (i.e., C₁-C₂₀ alkoxy), 1 to 12 carbon atoms (i.e., C₁-C₁₂ alkoxy), 1 to 10 carbon atoms (i.e., C₁-C₁₀ alkoxy), or 1 to 6 carbon atoms (i.e., C₁-C₆ alkoxy). Examples of a suitable alkoxy group may include methoxy (-O-CH₃ or -OMe), ethoxy (-OCH₂CH₃ or -OEt), and t-butoxy (-OC(CH₃)₃ or -O-tBu), but are not limited thereto.

"Alkenyl" has primary, secondary, tertiary and/or quaternary carbon atoms, includes straight-chain, branched and cyclic groups, or a combination thereof, and has hydrocarbon with one or more unsaturation regions, that is, carbon-carbon sp² double bonds. For example, the alkenyl group may have 2 to 20 carbon atoms (i.e., C₂-C₂₀ alkenyl), 2 to 12 carbon atoms (i.e., C₂-C₁₂ alkenyl), 2 to 10 carbon atoms (i.e., C₂-C₁₀ alkenyl), or 2 to 6 carbon atoms (i.e., C₂-C₆ alkenyl). Examples of a suitable alkenyl group may include vinyl (-CH=CH₂), allyl (-CH₂CH=CH₂) , cyclopentenyl (-C₅H₇), and 5-hexenyl (-CH₂CH₂CH₂CH₂CH=CH₂), but are not limited thereto.

"Alkynyl" has primary, secondary, tertiary and/or quaternary carbon atoms, includes straight-chain, branched and cyclic groups, or a combination thereof, and has hydrocarbon with one or more carbon-carbon sp triple bonds. For example, the alkynyl group may have 2 to 20 carbon atoms (i.e., C₂-C₂₀ alkynyl), 2 to 12 carbon atoms (i.e., C₂-C₁₂ alkynyl), 2 to 10 carbon atoms (i.e., C₂-C₁₀ alkynyl), or 2 to 6 carbon atoms (i.e., C₂-C₆ alkynyl). Examples of a suitable alkynyl group may include acetylenyl (-C=CH) and propynyl (-CH₂C≡CH), but are not limited thereto.

As used herein, the term "aryl" includes a substituted or unsubstituted monovalent or divalent aromatic hydrocarbon group, which is monocyclic, bicyclic or polycyclic where each ring atom is carbon. Preferably, an aryl ring is a 6- to 20-membered ring, a 6- to 14-membered ring, a 6- to 10-membered ring, or more preferably a 6-membered ring. The aryl group may be a polycyclic ring system having two or more cyclic rings where two or more carbons are common to two adjacent rings, in which one or more of the rings may be aromatic, and for example, the other cyclic ring may be cycloalkyl, cycloalkenyl, cycloalkynyl, aryl, heteroaryl, and/or heterocycloalkyl. Examples of the aryl group may include benzene, naphthalene, phenanthrene, anthracene, indene, indane, phenol, aniline, etc.

As used herein, the term "carbocyclylalkyl", "cycloalkylalkyl", or "(cycloalkyl)alkyl" refers to an alkyl group substituted with a carbocycle group or a cycloalkyl group.

As used herein, the term "carbocycle", "carbocyclyl", "carbocyclic", or "cycloalkyl" may be monocyclic, bicyclic, or polycyclic and refers to a non-aromatic saturated or unsaturated, monovalent or divalent ring where each ring atom is carbon. The cycloalkyl group may have 3 to 7 carbon atoms as a monocycle, 7 to 12 carbon atoms as a bicycle, and about 20 carbon atoms or less as a polycycle. Monocyclic cycloalkyl has 3 to 7 ring atoms, more typically 5 or 6 ring atoms. Bicyclic cycloalkyl may have 7 to 12 ring atoms, and may be a fused ring system, a spirocyclic ring system, or a bridged ring system. In an exemplary cycloalkyl group, atoms may be arranged in a bicyclo [4,5], [5,5], [5,6], or [6,6] system. In a specific embodiment, cycloalkyl contains 3 to 20 atoms, or 3 to 10 atoms, or more preferably 3 to 7 atoms. Examples of cycloalkyl may include cyclopropyl, cyclobutyl, cyclopentyl, cyclohexyl, cycloheptyl, cyclooctyl, etc. Unless otherwise specified, cycloalkyl may be substituted by one or more substituents described herein.

As used herein, the terms "heterocyclylalkyl" and "heterocycloalkyl" refer to an alkyl group substituted with a heterocycloalkyl group.

The terms "heterocyclyl", "heterocycle", "heterocyclic", and "heterocycloalkyl" refer to substituted or unsubstituted, monovalent or divalent, saturated or partially saturated non-aromatic ring structures, preferably 3- to 10-membered rings, more preferably 3- to 7-membered rings, in which the ring structure contains at least 1 heteroatom, preferably 1 to 4 heteroatoms, and more preferably 1 to 2 heteroatoms. The terms "heterocyclyl", "heterocycle", "heterocyclic", and "heterocycloalkyl" may also include a polycyclic ring system having two or more cyclic rings where two or more carbons are common to two adjacent rings, in which one or more of the rings may be heterocyclic, and for example, the other cyclic ring may be cycloalkyl, cycloalkenyl, cycloalkynyl, aryl, heteroaryl, and/or heterocyclyl. Bicyclic and polycyclic heterocyclic ring systems may be fused, bridged, or spiro ring systems. Substituted heterocycle includes, for example, a heterocyclic ring substituted with any substituent disclosed herein, including a carbonyl group. The heterocyclyl group includes, for example, piperidine, piperazine, pyrrolidine, morpholine, lactone, lactam, etc. Additional exemplary heterocyclo may include dihydropyridyl, dihydroindolyl, tetrahydropyridyl (piperidyl), tetrahydrothiophenyl, sulfur-oxidized tetrahydrothiophenyl, indolenyl, piperidinyl, 4-piperidinyl, pyrrolidinyl, 2-pyrrolidonyl, pyrrolinyl, tetrahydrofuranyl, tetrahydroquinolinyl, tetrahydroisoquinolinyl, decahydroquinolinyl, octahydroisoquinolinyl, 6H-1,2,5-thiadiazinyl, 2H,6H-1,5,2-dithiazinyl, pyranyl, chromenyl, xanthenyl, phenoxatinyl, 2H-pyrrolyl, 3H-indolyl, 4H-quinolizinyl, phthalazinyl, naphthyridinyl, quinoxalinyl, quinazolinyl, cinnolinyl, pteridinyl, 4aH-carbazolyl, carbazolyl, B-carbolinyl, phenanthridinyl, acridinyl, phenanthrolinyl, phenazinyl, phenothiazinyl, furazanyl, phenoxazinyl, isochromanyl, chromanyl, imidazolidinyl, imidazolinyl, pyrazolidinyl, pyrazolinyl, piperazinyl, methyl piperazinyl, quinuclidinyl, morpholinyl, and oxazolidinyl (each of these may be substituted or unsubstituted), but are not limited thereto.

"Heteroaryl" refers to a substituted or unsubstituted monovalent or divalent aromatic group that is monocyclic, bicyclic or polycyclic containing one or more heteroatoms in the ring. Non-limiting examples of a suitable heteroatom that may be contained in the aromatic ring may include oxygen, sulfur, and nitrogen. In a polycyclic heteroaryl ring system, the ring system has two or more cyclic rings where two or more carbons are common to two adjacent rings, in which one or more of the rings may be heteroaromatic, and for example, other cyclic rings may be cycloalkyl, cycloalkenyl, cycloalkynyl, aryl, heteroaryl, and/or heterocyclyl. The hetero group includes, for example, benzofuran, benzothiophene, pyrrole, furan, thiophene, imidazole, indole, isoindole, isoxazole, isothiazole, oxazole, thiazole, quinoline, isoquinoline, pyrazole, pyridine, pyrazine, pyridazine, pyrimidine, etc. (each of these may be substituted or unsubstituted).

As used herein, the terms "halo" and "halogen" mean halogen and include chloro, fluoro, bromo, and iodo.

"Amino" refers to a -NH₂ group.

"Carboxy" refers to a -C(O)OH group.

"Aldehyde" refers to a -CHO group.

The present disclosure relates to a compound of Chemical Formula I below, a solvate, a stereoisomer or a pharmaceutically acceptable salt thereof.

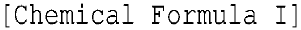

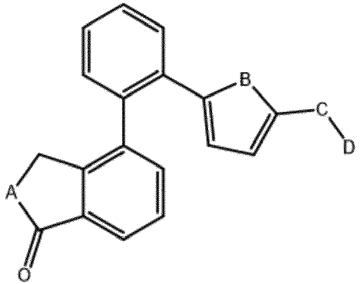

in which,
A and B are each independently any one selected from the group consisting of oxygen, nitrogen, and sulfur, and when A or B is nitrogen, the hydrogen bonded to nitrogen is substituted with an alkyl group,
C is any one selected from the group consisting of hydrogen, C₁₋₆ alkyl, and carbonyl, and
D is hydrogen, C₁₋₆ alkyl, -NH₂, -NH(C₁₋₆ alkyl), -N(C₁₋₆ alkyl) (C₁₋₆ alkyl), -NH (C₁₋₆ cycloalkyl), cycloalkyl, heterocycloalkyl, aryl or heteroaryl.

Specifically, in the present disclosure, a hydrogen bonding moiety of the -NH(C₁₋₆ alkyl), -N(C₁₋₆ alkyl) (C₁₋₆ alkyl), -NH(C₁₋₆ cycloalkyl), cycloalkyl, heterocycloalkyl, aryl, and heteroaryl may be substituted with at least one substituent selected from the group consisting of hydrogen, halogen, -OH, =O, -OC₁₋₆ alkyl, -C₁₋₆ alkyl, -C(=O)Ra, -C(=O)N(Ra)(Rb), -C₁₋₆ alkylC(=O)N(Ra) (Rb), -C₃H₄C(=O)N(Ra) (Rb), -C=CH, -C₃₋₆ cycloalkyl, -N(C₁₋₆ alkyl) (C₁₋₆ alkyl), -SO₂ (C₁₋₆ alkyl), phenyl, phenyl in which at least one of hydrogen or carbon is substituted with halogen, methyl piperazinyl, pyrazoyl in which any one of hydrogens is substituted with C₁₋₆ alkyl, pyrrolidonyl in which any one of hydrogens is substituted with halogenated phenyl, methyl pyrrolidonyl in which any one of hydrogens is substituted with halogenated phenyl, benzyl, benzyl in which at least one of hydrogen or carbon is substituted with nitrogen, and benzyl in which at least one of hydrogen or carbon is substituted with halogen, and the Ra and Rb may each independently be H, C₁₋₆ alkyl, C₃₋₆ cycloalkyl, and benzyl.

More specifically, in the present disclosure, the heterocycloalkyl of D may include at least one selected from the group consisting of pyrrolidinyl, piperidinyl, piperazinyl, methyl piperazinyl, morpholinyl, and piperazinonyl.

More specifically, in the present disclosure, the C may be either methyl or carbonyl.

Further, the present disclosure relates to a compound of Chemical Formula II below, a solvate, a stereoisomer or a pharmaceutically acceptable salt thereof. 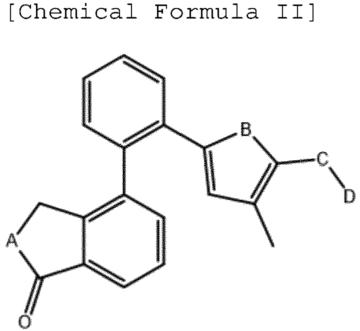 in which,
A and B are each independently any one selected from the group consisting of oxygen, nitrogen, and sulfur, and when A or B is nitrogen, the hydrogen bonded to nitrogen is substituted with an alkyl group,
C is hydrogen or C₁₋₆ alkyl,
D is hydrogen, C₁₋₆ alkyl, -NH₂, -NH(C₁₋₆ alkyl), -N(C₁₋₆ alkyl)(C₁₋₆ alkyl), cycloalkyl, heterocycloalkyl, aryl or heteroaryl,
the cycloalkyl, heterocycloalkyl, aryl, and heteroaryl may be substituted with at least one substituent selected from the group consisting of hydrogen, -OH, =O, -C₁₋₆ alkyl, -C(=O)Ra, -C(=O)N(Ra) (Rb), -C₁₋₆ alkylC(=O)N(Ra) (Rb), and benzyl in which at least one of hydrogen or carbon is substituted with halogen,
the heterocycloalkyl includes at least one selected from the group consisting of pyrrolidinyl, piperidinyl, piperazinyl, methyl piperazinyl, morpholinyl, and piperazinonyl, and
the Ra and Rb are each independently H or C₁₋₆ alkyl.

Specifically, in the present disclosure, the C may be methyl.

In addition, the present disclosure relates to a compound of Chemical Formula I selected from the group consisting of compounds represented by Chemical Formulas in Table 1 below, a solvate, a stereoisomer, or a pharmaceutically acceptable salt thereof, but is not limited thereto.

**[Table 1]**

| | Chemical Formula |
|---|---|
| Compound 1 | 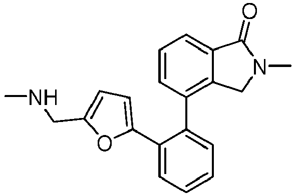 |
| Compound 2 | 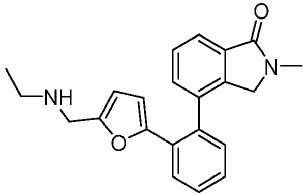 |
| Compound 3 | 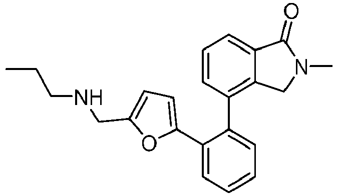 |
| Compound 4 | 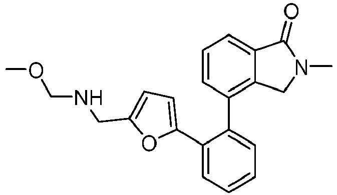 |
| Compound 5 | 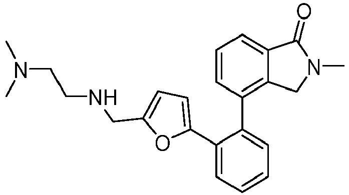 |
| Compound 6 | 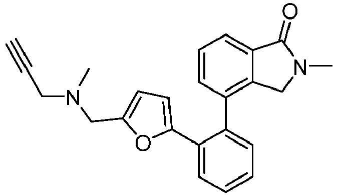 |
| Compound 7 | 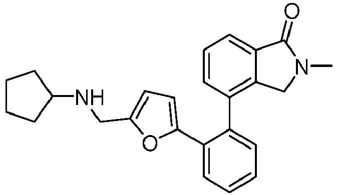 |
| Compound 8 | 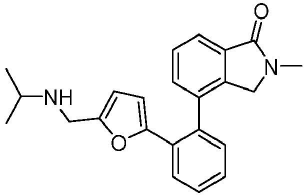 |
| Compound 9 | 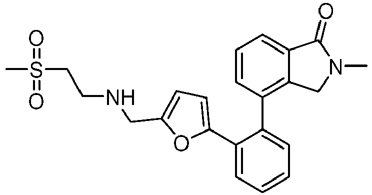 |
| Compound 10 | 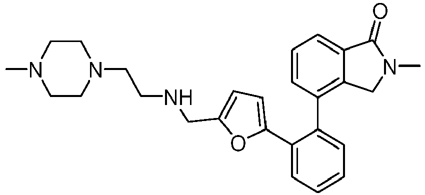 |
| Compound 11 | 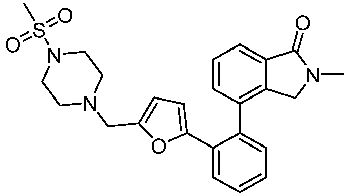 |
| Compound 12 | 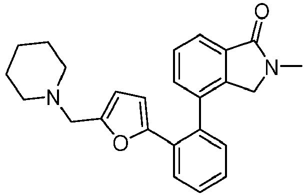 |
| Compound 13 | 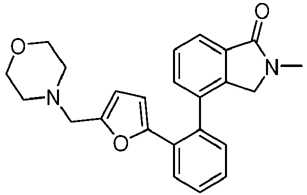 |
| Compound 14 | 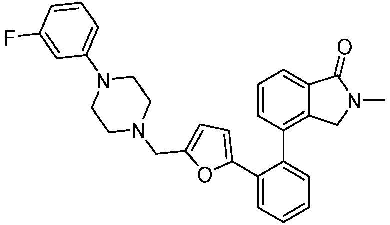 |
| Compound 15 | 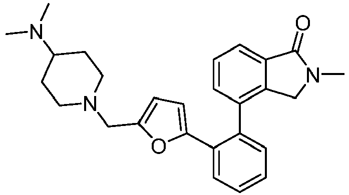 |
| Compound 16 | 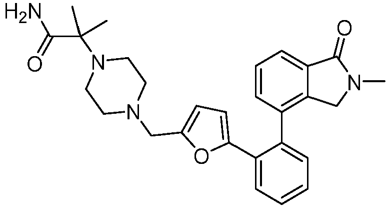 |
| Compound 17 | 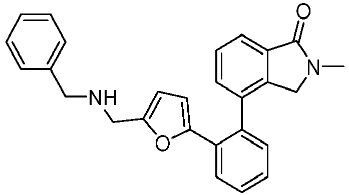 |
| Compound 18 | 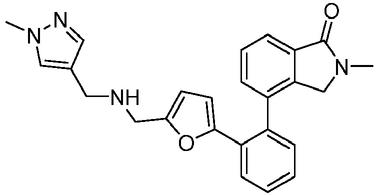 |
| Compound 19 | 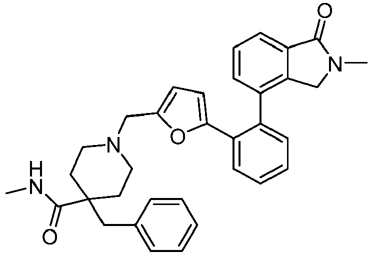 |
| Compound 20 | 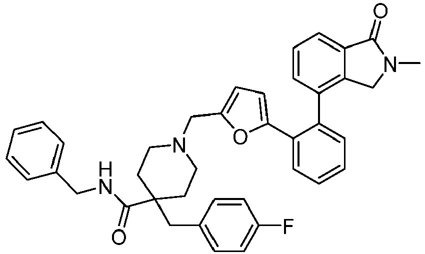 |
| Compound 21 | 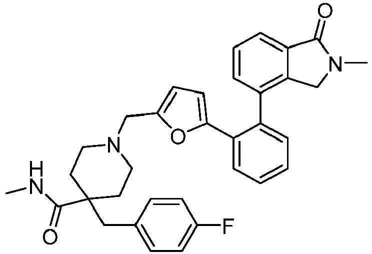 |
| Compound 22 | 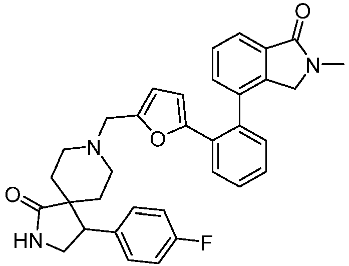 |
| Compound 23 | 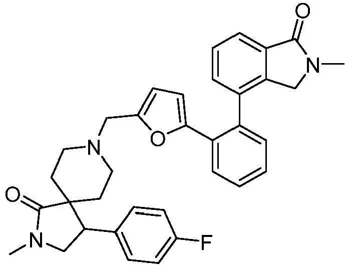 |
| Compound 24 | 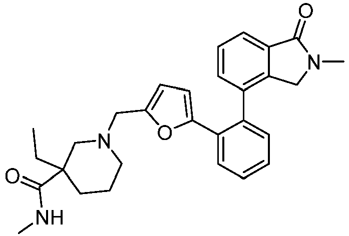 |
| Compound 25 | 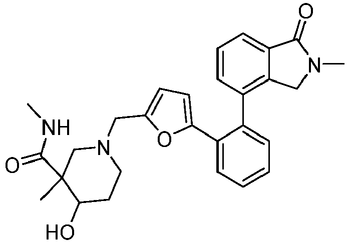 |
| Compound 26 | 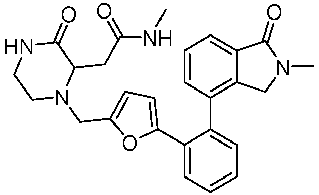 |
| Compound 27 | 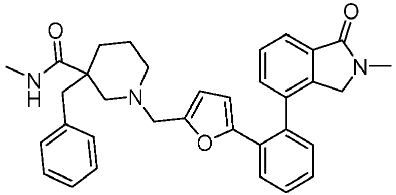 |
| Compound 28 | 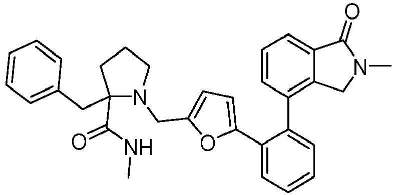 |
| Compound 29 | 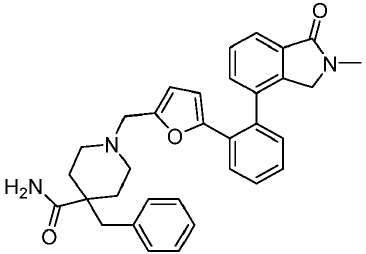 |
| Compound 30 | 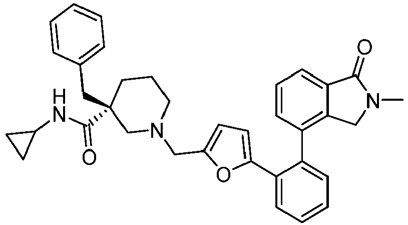 |
| Compound 31 | 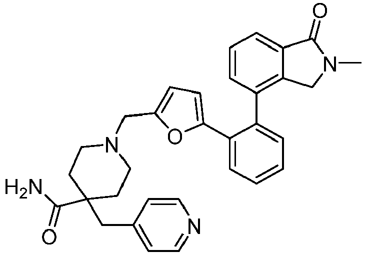 |
| Compound 32 | 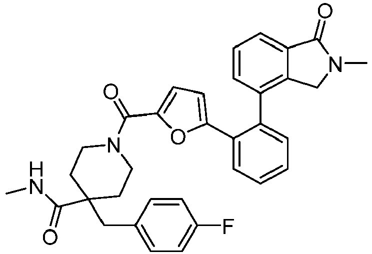 |
| Compound 33 | 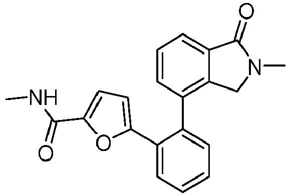 |
| Compound 34 | 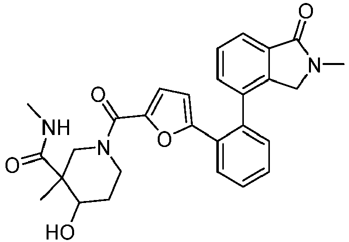 |
| Compound 35 | 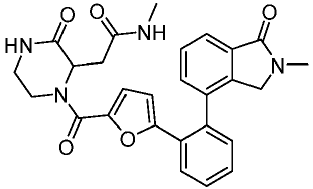 |
| Compound 36 | 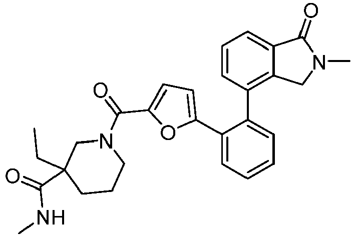 |
| Compound 37 | 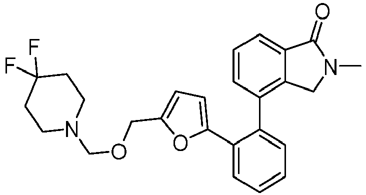 |

In addition, the present disclosure relates to a compound of Chemical Formula II selected from the group consisting of compounds represented by Chemical Formulas in Table 2 below, a solvate, a stereoisomer, or a pharmaceutically acceptable salt thereof, but is not limited thereto.

**[Table 2]**

| | Chemical Formula |
|---|---|
| Compound 38 | 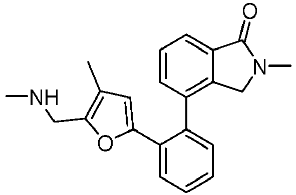 |
| Compound 39 | 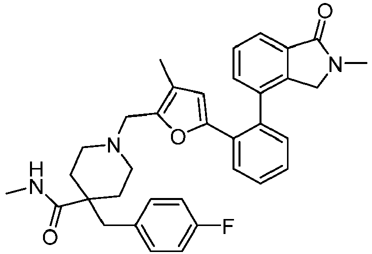 |
| Compound 40 | 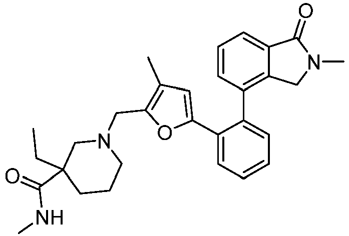 |
| Compound 41 | 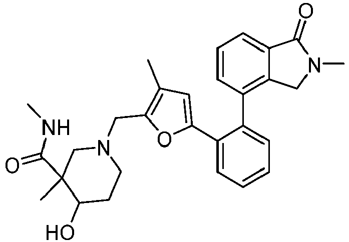 |
| Compound 42 | 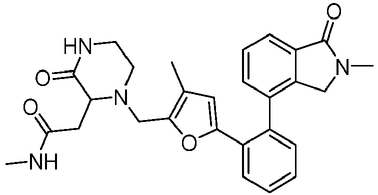 |

Further, the present disclosure relates to a pharmaceutical composition for preventing or treating ophthalmic diseases, including as an active ingredient the compound of Chemical Formula I or II, a solvate, a stereoisomer or a pharmaceutically acceptable salt thereof.

The ophthalmic disease includes endophthalmitis, keratitis, conjunctivitis, keratoconjunctivitis, uveitis, blepharitis, scleritis, iritis, glaucoma, retinal degeneration, retinitis pigmentosa, retinal detachment, retinal pigment epithelium detachment, retinal break, diabetic retinopathy, retinopathy of prematurity, polypoidal choroidal vasculopathy, ischemic proliferative retinopathy, cone dystrophy, proliferative vitreoretinopathy, retinal artery occlusion, retinal vein occlusion, Leber's optic atrophy, corneal neovascularization, retina choroidal neovascularization, wet and dry macular degeneration, and age-related macular degeneration, but is not limited thereto. Preferably, the ophthalmic disease may be diabetic retinopathy, glaucoma, uveitis, wet and dry macular degeneration, or age-related macular degeneration.

As used herein, the term "treating" or "treatment" means inhibiting diseases, for example, inhibiting a disease, condition or disorder in a subject experiencing or exhibiting pathology or symptoms of the disease, condition or disorder, that is, preventing additional occurrence of pathology and/or symptoms, or improving diseases, for example, improving a disease, condition or disorder in a subject experiencing or exhibiting pathology or symptoms of the disease, condition or disorder, that is, reversing pathology and/or symptoms, such as reducing disease severity.

As used herein, the term "preventing" or "prevention" refers to preventing a disease, for example, preventing a disease, a condition, or a disorder in a subject who may have a disposition to the disease, condition, or disorder, but has not yet experienced or exhibited pathology or symptoms of the disease.

In an embodiment, the pharmaceutical composition may include conventional pharmaceutically acceptable carriers, excipients, or additives. The pharmaceutical composition may be formulated according to conventional methods, and may be prepared in various oral dosage forms such as tablets, pills, powders, capsules, syrups, emulsions, and microemulsions, or parenteral dosage forms such as intramuscular, intravenous, or subcutaneous administration.

When the pharmaceutical composition is prepared in the form of an oral formulation, examples of additives or carriers to be used may include cellulose, calcium silicate, corn starch, lactose, sucrose, dextrose, calcium phosphate, stearic acid, magnesium stearate, calcium stearate, gelatin, talc, surfactants, suspending agents, emulsifiers, diluents, etc. When the pharmaceutical composition of the present disclosure is prepared in the form of an injection, the additives or carriers may include water, a saline solution, an aqueous glucose solution, a simulated aqueous sugar solution, alcohol, glycol, ether (e.g., polyethylene glycol 400), oils, fatty acid, fatty acid ester, glyceride, a surfactant, a suspending agent, an emulsifier, etc.

The dose of the pharmaceutical composition is an effective amount for the treatment or prevention of a subject or patient, and may be administered orally or parenterally depending on the purpose. When administered orally, the dose may be administered in an amount of 3 to 20 mg, more specifically 5 to 10 mg per 1 kg of body weight daily based on the active ingredient, and when administered parenterally, the dose may be administered in an amount of 3 to 20 mg, more specifically 5 to 10 mg per 1 kg of body weight daily based on the active ingredient so that it may be administered in one to several divided doses. It should be understood that the dose for a specific subject or patient needs to be determined depending on various related factors such as the weight, age, sex, health condition of a patient, diet, administration time, administration method, the severity of a disease, etc., and may be adjusted appropriately by experts, and the dose is not intended to limit the scope of the present disclosure in any way. Physicians or veterinarians having ordinary skills in the relevant art may easily determine and prescribe an effective amount of the required pharmaceutical composition. For example, the physicians or veterinarians may start the dose of the compound of the present disclosure used in the pharmaceutical composition at a level lower than that required to achieve a desired therapeutic effect and gradually increase the dose until the desired effect is achieved.

Specifically, the compound of Chemical Formula I or II of the present disclosure, the solvate, the stereoisomer or the pharmaceutically acceptable salt thereof may prevent or treat macular degeneration by inhibiting angiogenesis in the eye, and the macular degeneration may include wet and dry macular degeneration, and age-related macular degeneration, but is not limited thereto. In addition, the compound of Chemical Formula I or II of the present disclosure, the solvate, the stereoisomer or the pharmaceutically acceptable salt thereof may be applied even to various ophthalmic diseases such as diabetic retinopathy, glaucoma, and uveitis.

In addition, from the experimental results of Examples, the novel compound of the present disclosure alleviates inflammation caused by various epigenetic changes, such as inhibiting BRD proteins, and inhibits retinal degeneration, thereby exhibiting excellent therapeutic effects on various ophthalmic diseases such as diabetic retinopathy, glaucoma, uveitis, age-related macular degeneration, wet and dry macular degeneration, etc.

The present disclosure relates to a pharmaceutical composition including a novel compound for the prevention or treatment of various ophthalmic diseases such as diabetic retinopathy, glaucoma, uveitis, wet and dry macular degeneration, and age-related macular degeneration.

Hereinafter, the present disclosure will be described in detail with reference to Examples for understanding. However, the following Examples are merely illustrative of the contents of the present disclosure, and the scope of the present disclosure is not limited to the following Examples. Examples of the present disclosure will be provided for more completely explaining the present disclosure to those skilled in the art.

### [Preparation Example]

In the present disclosure, the structures of compounds were confirmed by nuclear magnetic resonance (NMR) and mass spectrometry (MS). The NMR was measured with a Bruker Avance-400 or Bruker Avance 300 instrument. Solvents for the measurement were deuterium substituted-dimethyl sulfoxide (DMSO-d6), deuterium substituted-chloroform (CDCl₃) and deuterium substituted-methanol (CD₃OD), and an internal standard was tetramethylsilane (TMS).

High-performance liquid chromatography (HPLC) was performed by flash chromatography or column chromatography.

Thin layer chromatography (TLC) was performed on a silica gel plate. 1000 mesh silica gel was used for thin layer chromatography. In addition, the size of a silica gel plate used for TLC was 20 to 25 µm, and the size of a silica gel plate used for product purification was 40 to 45 µm.

For visualization, ultraviolet light, iodine, and potassium permanganate were used in water.

Known starting substances of the present disclosure may be prepared by conventional synthetic methods in the art, or purchased from Sigma-Aldrich, TCI, Wako, Kanto, Fluorchem, Acros, Alfa, Fluka, Combi-Blocks, Dae-Jung, etc.

Compounds 1 to 42 of the present disclosure were prepared according to the following Preparation Examples and Examples.

### <Preparation Example 1> Preparation of 5-(2-(2-methyl-1-oxoisoindoline-4-yl)phenyl)furan-2-carboaldehyde

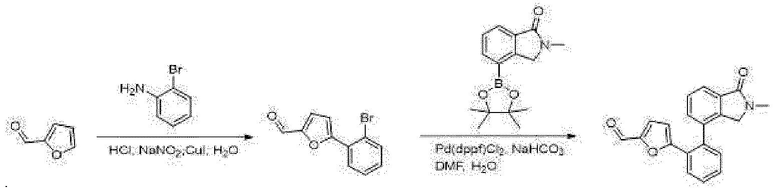

### Preparation Example 1-1) Preparation of 5-(2-bromophenyl)furan-2-carboaldehyde

2-bromoaniline (3.36 g, 35.0 mmol) was dissolved in a 2N-HCl aqueous solution (50 ml), added dropwise with cuprous iodide (666 mg, 3.50 mmol) and then stirred at 0°C. At the same temperature, sodium nitrite (4.83 g, 70.0 mmol) was slowly added dropwise and stirred for 1 hour, and then added with furan-2-carbaldehyde (5.04 g, 52.5 mmol) and stirred at room temperature for 12 hours. The mixture was added with ethyl acetate (200 ml), washed with water (200 ml, twice), dried over anhydrous magnesium sulfate, and then the filtered filtrate was distilled under reduced pressure. The filtrate was separated using silica gel column chromatography to obtain the following compounds (2.00 g, 7.96 mmol).
¹H-NMR (400MHz, DMSO-d₆) δ 9.68(s, 1H), 7.66(m, 2H), 7.73(d, 1H), 7.52(t, 1H), 7.44(t, 1H), 7.32(s, 1H)

### Preparation Example 1-2) Preparation of 5-(2-(2-methyl-1-oxoisoindoline-4-yl)phenyl)furan-2-carboaldehyde

The compound (2.00 g, 7.96 mmol) obtained in Preparation Example 1-1) was dissolved in dimethylformamide (40 ml), added with sodium bicarbonate (669 mg, 7.96 mmol) and water (10 ml), and then stirred. The mixture was added with (1,1'-bis(diphenylphosphino)ferrocene)palladium(II) chloride (0.796 mmol, 582.4 mg) and 2-methyl-4-(4,4,5,5-tetramethyl-1,3,2-dioxaboran-2-yl)isoindoline-1-one (2.61 g, 9.55 mmol), and stirred at 80 to 90°C for 1 hour. When the reaction was completed, the mixture was cooled to room temperature, added with water, and then extracted with ethyl acetate (100 ml). The extracted solution was dried with anhydrous magnesium sulfate and concentrated under reduced pressure to obtain a residue. The residue was separated by silica gel column chromatography to obtain the following compounds (1.40 g, 4.41 mmol).
¹H-NMR (400MHz, DMSO-d₆) δ 9. 69 (s, 1H), 8.05 (d, 1H), 7.96(d, 2H), 7.94(d, 1H), 7.69(t, 1H), 7.66(d, 1H), 7.61(t, 1H), 7.32(d, 1H), 4.22(brs, 2H), 3.26(s, 3H)

### <Preparation Example 2> Preparation of 4-(2-(5-(chloromethyl)furan-2-yl)phenyl)-2-methylisoindoline-1-one

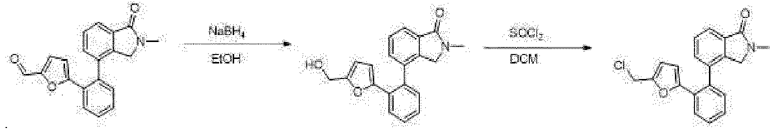

### Preparation Example 2-1) Preparation of 4-(2-(5-(hydroxymethyl)furan-2-yl)phenyl)-2-methylisoindoline-1-one

5-(2-(2-methyl-1-oxoisoindoline-4-yl)phenyl)furan-2-carboaldehyde (10.0 g, 31.5 mmol) synthesized in Preparation Example 1 was added to anhydrous ethanol (100 ml) and dissolved, and added dropwise with sodium borohydride (1.79 g, 47.2 mmol) at 0°C, and stirred. It was confirmed that the mixture was stirred for 1 hour at the same temperature to complete the reaction, and the reaction was terminated with a 10% sodium hydroxide solution. The ethanol was concentrated under reduced pressure, and the residue was extracted three times by adding dichloromethane and water. The extracted solution was dried with anhydrous magnesium sulfate and concentrated under reduced pressure to obtain a residue.

### Preparation Example 2-2) Preparation of 4-(2-(5-(chloromethyl)furan-2-yl)phenyl)-2-methylisoindoline-1-one

4-(2-(5-(hydroxymethyl)furan-2-yl)phenyl)-2-methylisoindoline-1-one (7.0 g, 21.9 mmol) synthesized in Preparation Example 2-1) was dissolved in dichloromethane (100 ml), slowly added dropwise with thionyl chloride (2.38 ml, 32.8 mmol) at 0°C and then stirred at the same temperature for 1 hour. When the reaction was completed, the reaction was terminated by adding water (30 ml), and the mixture was extracted three times by adding dichloromethane and water. The extracted solution was dried with anhydrous magnesium sulfate and concentrated under reduced pressure to obtain a residue.
¹H-NMR (400MHz, DMSO-d₆) δ 8.05 (d, 1H), 7.96 (d, 2H), 7.94(d, 1H), 7.69(t, 1H), 7.66(d, 1H), 7.61(t, 1H), 7.32(d, 1H), 4.49(s, 2H), 4.22(brs, 2H), 3.27(s, 3H)

### <Preparation Example 3> Preparation of 3-methyl-5-(2-(2-methyl-1-oxoisoindoline-4-yl)phenyl)furan-2-carboaldehyde

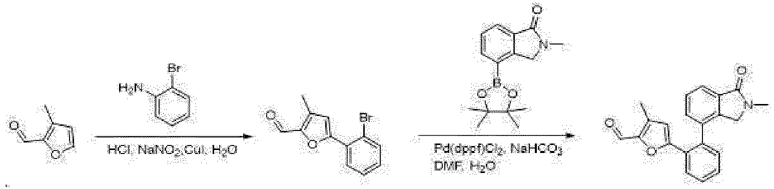

3-methyl-5-(2-(2-methyl-1-oxoisoindoline-4-yl)phenyl)furan-2-carboaldehyde was prepared in the same manner as in Preparation Example 1 using 3-methyl-2-furancarboxaldehyde.
¹H-NMR (400MHz, DMSO-d₆) δ 9.68(s, 1H), 8. 05 (d, 1H), 7.96(d, 2H), 7.94(d, 1H), 7.69(t, 1H), 7.60(d, 2H), 4.22(s, 2H), 3.27(s, 3H), 2.33(s, 3H)

### Examples

### Example 1: Synthesis of 2-methyl-4-(2-(5-((methylamino)methyl)furan-2-yl)phenyl)isoindoline-1-one

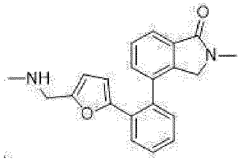

5-(2-(2-methyl-1-oxoisoindoline-4-yl)phenyl)furan-2-carboaldehyde (80 mg, 0.252 mmol) obtained in Preparation Example 1-2) was added to dichloromethane (5 ml), added sequentially with sodium triacetoxyborohydride (80.1 mg, 0.378 mmol), acetic acid (10 µl), and methylamine hydrochloride (25.5 mg, 0.378 mmol), and then stirred at room temperature for 12 hours. When the reaction was completed, the mixture was added with water and extracted with dichloromethane (20 ml). The extracted solution was dried with anhydrous magnesium sulfate and concentrated under reduced pressure to obtain a residue. The residue was separated by silica gel column chromatography to obtain 2-methyl-4-(2-(5-((methylamino)methyl)furan-2-yl)phenyl)isoindoline-1-one (16.3 mg, 0.478 mmol).
¹H-NMR (400MHz, DMSO-d₆) δ 8.04 (d, 1H), 7.97 (d, 2H), 7.94(d, 1H), 7.70(t, 1H), 7.66(d, 1H), 7.61(t, 1H), 7.32(d, 1H), 6.29(d, 1H), 4.16(m, 1H), 4.22(brs, 2H), 3.65(d, 2H), 3.27(s, 3H), 3.25(d, 3H)
MS(ESI+) m/z 333 (M+H)⁺

### Examples 2 to 26 below were synthesized in the same manner as Example 1, or prepared using appropriate reactants in consideration of the structures of the compounds to be prepared.

### Example 2 : Synthesis of 2-methyl-4-(2-(5-((ethylamino)methyl)furan-2-yl)phenyl)isoindoline-1-one

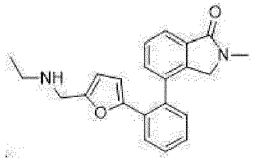

¹H-NMR (400MHz, DMSO-d₆) δ 8.03 (d, 1H), 7.95 (d, 2H), 7.94(d, 1H), 7.70(t, 1H), 7.65(d, 1H), 7.61(t, 1H), 7.32(d, 1H), 6.29(d, 1H), 4.16(m, 1H), 4.22(brs, 2H), 3.65(d, 2H), 3.27(s, 3H), 2.66(m, 2H), 1.12(t, 3H)
MS(ESI+) m/z 347 (M+H)⁺

### Example 3 : Synthesis of 2-methyl-4-(2-(5-((propylamino)methyl)furan-2-yl)phenyl)isoindoline-1-one

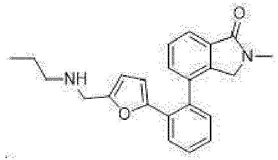

¹H-NMR (400MHz, DMSO-d₆) δ 8.03 (d, 1H), 7.95 (d, 2H), 7.94(d, 1H), 7.70(t, 1H), 7.65(d, 1H), 7.61(t, 1H), 7.32(d, 1H), 6.29(d, 1H), 4.22(brs, 2H), 4.16(m, 1H), 3.65(d, 2H), 3.27(s, 3H), 2.66(m, 2H), 1.12(t, 3H)
MS(ESI+) m/z 361 (M+H)⁺

### Example 4: Synthesis of 2-methyl-4-(2-(5-((methoxymethylamino)methyl)furan-2-yl)phenyl)isoindoline-1-one

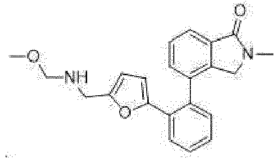

¹H-NMR (400MHz, DMSO-d₆) δ 8. 05 (d, 1H), 7.96(d, 2H), 7.94(d, 1H), 7.70(t, 1H), 7.65(d, 1H), 7.61(t, 1H), 7.32(d, 1H), 6.29(d, 1H), 4.63(d, 2H), 4.16(m, 1H), 4.21(brs, 2H), 3.65(d, 2H), 3.30(s, 3H), 3.27(s, 3H)
MS(ESI+) m/z 363 (M+H)⁺

### Example 5 : Synthesis of 2-methyl-4-(2-(5-((dimethylaminoethylamino)methyl)furan-2-yl)phenyl)isoindoline-1-one

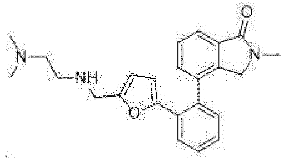

¹H-NMR (400MHz, DMSO-d₆) δ 8.05 (d, 1H), 7.96 (d, 2H), 7.94(d, 1H), 7.70(t, 1H), 7.65(d, 1H), 7.61(t, 1H), 7.32(d, 1H), 6.28(d, 1H), 4.22(brs, 2H), 4.16(m, 1H), 3.66(d, 2H), 3.27(s, 3H), 2.50(m, 2H), 2.39(d, 2H), 2.21(s, 6H)
MS(ESI+) m/z 390 (M+H)⁺

### Example 6: Synthesis of 2-methyl-4-(2-(5-((methyl(prop-2-yn-1-yl)amino))methyl)furan-2-yl)phenyl)isoindoline-1-one

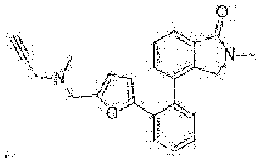

¹H-NMR (400MHz, DMSO-d₆) δ 8.05 (d, 1H), 7.96 (d, 2H), 7.94(d, 1H), 7.70(t, 1H), 7.65(d, 1H), 7.61(t, 1H), 7.32(d, 1H), 6.29(d, 1H), 4.22(brs, 2H), 3.87(s, 2H), 3.76(s, 2H), 3.27(s, 3H), 3.08(s, 1H), 2.25(s, 3H)
MS(ESI+) m/z 371 (M+H)⁺

### Example 7: Synthesis of 2-methyl-4-(2-(5-((cyclopentylamino)methyl)furan-2-yl)phenyl)isoindoline-1-one

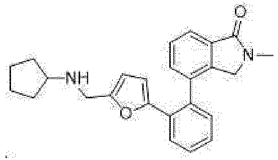

¹H-NMR (400MHz, DMSO-d₆) δ 8.05 (d, 1H), 7.96(d, 2H), 7.94(d, 1H), 7.70(t, 1H), 7.65(d, 1H), 7.61(t, 1H), 7.32(d, 1H), 6.29(d, 1H), 4.22(brs, 2H), 4.16(brs, 1H), 3.66(d, 2H), 3.27(s, 3H), 2.64(m, 1H), 1.72-1.47(m, 4H), 1.73-1.63(m, 4H)
MS(ESI+) m/z 387 (M+H)⁺

### Example 8 : Synthesis of 2-methyl-4-(2-(5-((isopropylamino)methyl)furan-2-yl)phenyl)isoindoline-1-one

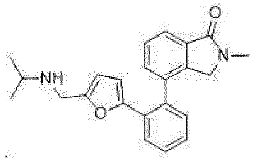

¹H-NMR (400MHz, DMSO-d₆) δ 8.05 (d, 1H), 7.96(d, 2H), 7.94(d, 1H), 7.70(t, 1H), 7.65(d, 1H), 7.61(t, 1H), 7.32(d, 1H), 6.29(d, 1H), 4.22(brs, 2H), 4.15(brs, 1H), 3.65(d, 2H), 3.27(s, 3H), 2.83(m, 1H), 1.06(s, 6H)
MS(ESI+) m/z 361 (M+H)⁺

### Example 9: Synthesis of 2-methyl-4-(2-(5-(((methylsulfonyl)ethyl)amino)methyl)furan-2-yl)phenyl)isoindoline-1-one

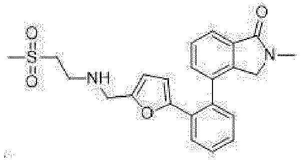

¹H-NMR (400MHz, DMSO-d₆) δ 8. 05 (d, 1H), 7.96(d, 2H), 7.94(d, 1H), 7.70(t, 1H), 7.65(d, 1H), 7.61(t, 1H), 7.32(d, 1H), 6.29(d, 1H), 4.22(brs, 2H), 4.15(brs, 1H), 3.65(d, 2H), 3.53(d, 2H), 3.27(s, 3H), 2.80(s, 3H)
MS(ESI+) m/z 425 (M+H)⁺

### Example 10: Synthesis of 2-methyl-4-(2-(5-(((2-(4-methylpiperazine-1-yl)ethyl)amino)methyl)furan-2-yl)phenyl)isoindoline-1-one

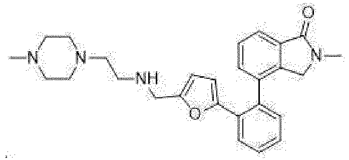

¹H-NMR (400MHz, DMSO-d₆) δ 8.05 (d, 1H), 7.96 (d, 2H), 7.94(d, 1H), 7.70(t, 1H), 7.65(d, 1H), 7.61(t, 1H), 7.32(d, 1H), 6.29(d, 1H), 4.22(brs, 2H), 4.16(brs, 1H), 3.65(d, 2H), 3.27(s, 3H), 2.50(m, 2H), 2.39(t, 2H), 2.27-2.29(m, 4H), 2.29-2.31(m, 4H), 2.14(s, 3H)
MS(ESI+) m/z 445 (M+H)⁺

### Example 11: Synthesis of 2-methyl-4-(2-(5-((4-methylsulfonyl)piperazine-1-yl)methyl))furan-2-yl)phenyl)isoindoline-1-one

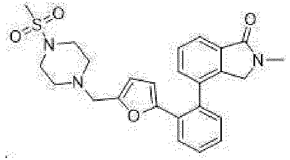

¹H-NMR (400MHz, DMSO-d₆) δ 8.05 (d, 1H), 7.96 (d, 2H), 7.94(d, 1H), 7.70(t, 1H), 7.65(d, 1H), 7.61(t, 1H), 7.32(d, 1H), 6.29(d, 1H), 4.22(brs, 2H), 3.76(S, 2H), 3.27(s, 3H), 2.45(m, 4H), 2.34(m, 4H), 2.87(s, 3H)
MS(ESI+) m/z 466 (M+H)⁺

### Example 12: Synthesis of 2-methyl-4-(2-(5-(piperidine-1-yl methyl)furan-2-yl)phenyl)isoindoline-1-one

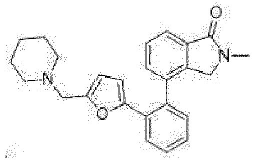

¹H-NMR (400MHz, DMSO-d₆) δ 8.05 (d, 1H), 7.96(d, 2H), 7.94(d, 1H), 7.70(t, 1H), 7.65(d, 1H), 7.61(t, 1H), 7.32(d, 1H), 6.29(d, 1H), 4.22(brs, 2H), 3.76(s, 2H), 3.27(s, 3H), 2.39-2.41(m, 4H), 1.43-1.42(m, 4H), 1.30(m, 2H)
MS(ESI+) m/z 387 (M+H)⁺

### Example 13: Synthesis of 2-methyl-4-(2-(5-(morpholinomethyl)furan-2-yl)phenyl)isoindoline-1-one

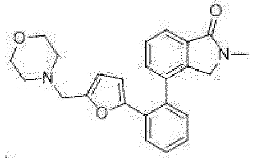

¹H-NMR (400MHz, DMSO-d₆) δ 8.05 (d, 1H), 7.96 (d, 2H), 7.94(d, 1H), 7.70(t, 1H), 7.65(d, 1H), 7.61(t, 1H), 7.32(d, 1H), 6.29(d, 1H), 4.22(brs, 2H), 3.76(s, 2H), 3.27(s, 3H), 3.50-3.52(m, 4H), 2.41-2.24(m, 4H)
MS(ESI+) m/z 389 (M+H)⁺

### Example 14: Synthesis of 2-methyl-4-(2-(5-(((4-(3-fluorophenyl)piperazine-1-yl)methyl))furan-2-yl)phenyl)isoindoline-1-one

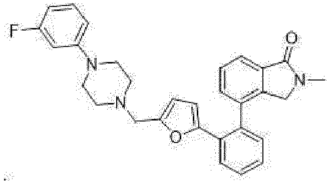

¹H-NMR (400MHz, DMSO-d₆) δ 8. 05 (d, 1H), 7.96(d, 2H), 7.94(d, 1H), 7.70(t, 1H), 7.65(d, 1H), 7.61(t, 1H), 7.32(d, 1H), 7.12(t, 1H), 6.83(s, 1H), 6.71(d, 1H), 6.44(d, 1H), 6.29(d, 1H), 4.22(brs, 2H), 3.76(d, 2H), 3.27(s, 3H), 3.20-3.25(m, 4H), 2.45-2.43(m, 4H)
MS(ESI+) m/z 482 (M+H)⁺

### Example 15: Synthesis of 2-methyl-4-(2-(5-((4-(dimethylamino)piperidine-1-yl)methyl)furan-2-yl)phenyl)isoindoline-1-one

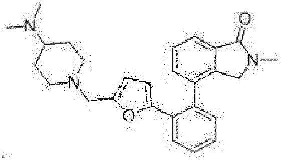

¹H-NMR (400MHz, DMSO-d₆) δ 8.05 (d, 1H), 7.96 (d, 2H), 7.94(d, 1H), 7.70(t, 1H), 7.65(d, 1H), 7.61(t, 1H), 7.32(d, 1H), 6.29(d, 1H), 4.22(brs, 2H), 3.76(s, 2H), 3.27(s, 3H), 2.63(t, 1H), 2.51-2.49(m, 4H), 1.65-1.55(m, 4H), 2.26(S, 6H),
MS(ESI+) m/z 431 (M+H)⁺

### Example 16: Synthesis of 2-methyl-4-(2-(5-(((4-(pivaloamidyl)piperazine-1yl)methyl)furan-2-yl)phenyl)isoindoline-1-one

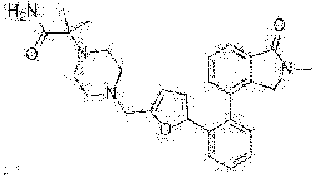

¹H-NMR (400MHz, DMSO-d₆) δ 8.05 (d, 1H), 7.96 (d, 2H), 7.94(d, 1H), 7.70(t, 1H), 7.65(d, 1H), 7.61(t, 1H), 7.32(d, 1H), 6.94(brs, 2H), 6.29(d, 1H), 4.22(brs, 2H), 3.76(d, 2H), 3.27(s, 3H), 2.32-2.20(m, 8H), 1.54(s, 6H)
MS(ESI+) m/z 473 (M+H)⁺

### Example 17: Synthesis of 2-methyl-4-(2-(5-((benzylamino)methyl)furan-2-yl)phenyl)isoindoline-1-one

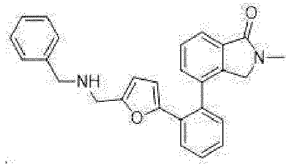

¹H-NMR (400MHz, DMSO-d₆) δ 8.05 (d, 1H), 7.96(d, 2H), 7.94(d, 1H), 7.70(t, 1H), 7.65(d, 1H), 7.61(t, 1H), 7.35(d, 2H), 7.32(m, 3H), 7.29(t, 1H), 6.29(d, 1H), 5.00(brs, 1H), 4.22(brs, 2H), 3.76(d, 2H), 3.66(d, 2H), 3.27(s, 3H)
MS(ESI+) m/z 409 (M+H)⁺

### Example 18: Synthesis of 2-methyl-4-(2-(5-((((1-methyl-1H-pyrazole-4yl)methyl)amino)methyl)furan-2-yl)phenyl)isoindoline-1-one

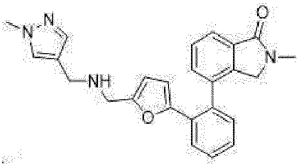

¹H-NMR (400MHz, DMSO-d₆) δ 8.05 (d, 1H), 7.96 (d, 2H), 7.94(d, 1H), 7.70(t, 1H), 7.65(d, 1H), 7.61(t, 1H), 7.47(s, 1H), 7.37(s, 1H), 7.32(d, 1H), 6.29(d, 1H), 5.00(brs, 1H), 4.22(brs, 2H), 3.94(s, 3H), 3.76(d, 2H), 3.66(d, 2H), 3.27(s, 3H)
MS(ESI+) m/z 413 (M+H)⁺

### Example 19: Synthesis of 2-methyl-4-(2-(5-((((4-benzyl-4'-methyl carboxamidyl)piperidine)methyl)furan-2-yl)phenyl)isoindoline-1-one

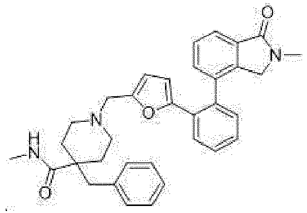

¹H-NMR (400MHz, DMSO-d₆) δ 8.05 (d, 1H), 7.96(d, 2H), 7.94(d, 1H), 7.70(t, 1H), 7.65(d, 1H), 7.61(t, 1H), 7.51(t, 1H), 7.32(d, 1H), 7.21(d, 2H), 7.23(m, 2H), 7.19(m, 1H), 6.29(d, 1H), 4.22(brs, 2H), 3.76(s, 2H), 3.27(s, 3H), 2.80(d, 3H), 2.71(s, 2H), 2.45(m, 4H), 1.80-1.67(m, 4H)
MS(ESI+) m/z 534 (M+H)⁺

### Example 20: Synthesis of 2-methyl-4-(2-(5-((((4-(4-fluorobenzyl)-4'-benzyl carboxamidyl)piperidine)methyl)furan-2-yl)phenyl)isoindoline-1-one

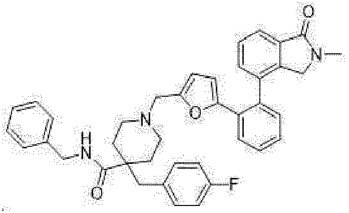

¹H-NMR (400MHz, DMSO-d₆) δ 8.87 (t, 1H), 8.05 (d, 1H), 7.96(d, 2H), 7.70(t, 1H), 7.65(d, 1H), 7.51(t, 1H), 7.23-7.30(m, 5H), 7.32(d, 1H), 7.21(d, 2H), 7.23(m, 2H), 7.19(m, 1H), 6.29(d, 1H), 4.24(t, 2H), 4.22(brs, 2H), 3.76(s, 2H), 3.27(s, 3H), 2.71(s, 2H), 2.51-2.45(m, 4H), 1.80-1.67(m, 4H)
MS(ESI+) m/z 628 (M+H)⁺

### Example 21: Synthesis of 2-methyl-4-(2-(5-(((4-(4-fluorobenzyl)-4'-carboxamidyl)piperidine)methyl)furan-2-yl)phenyl)isoindoline-1-one

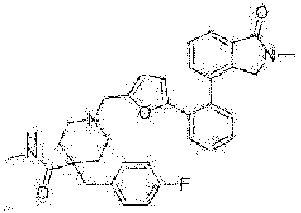

### [Step 1] Preparation of 4-(4-fluorobenzyl)-N-methylpiperidine-4-carboxamide hydrochloric acid

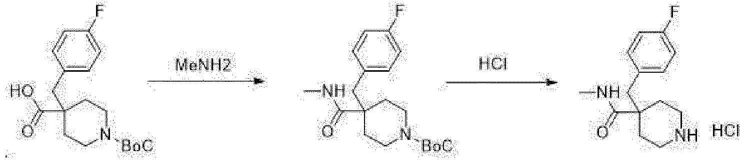

1-(tert-butoxycarbonyl)-4-(4-fluorobenzyl)piperidine-4-carboxylic acid (5.0 g, 14.8 mmol) was dissolved in anhydrous dimethylformamide, added with N,N,N',N'-tetramethyl-O-(1H-benzotriazole-1-yl)euronium hexafluorophosphate (HBTU) (8.42 g, 22.2 mmol) and N-diethyl-N-isopropyl amine (48.8 mmol), and then stirred at room temperature for 30 minutes. The reaction solution was added with methylamine hydrochloric acid (59.2 mmol) and stirred at 70°C for 12 hours. When the reaction was completed, the mixture was cooled to room temperature, added with water, and then extracted with ethyl acetate (200 ml). The extracted solution was dried with anhydrous magnesium sulfate and concentrated under reduced pressure to obtain a residue. The residue was dissolved in a 1N-HCl aqueous solution (100 ml) and stirred at 40°C for 4 hours. When the reaction was completed, the mixture was cooled to room temperature, added with water, and then extracted with ethyl acetate (200 ml, twice). The extracted solution was dried with anhydrous magnesium sulfate and concentrated under reduced pressure to obtain a residue. The residue was separated by silica gel column chromatography to obtain a 4-(4-fluorobenzyl)-N-methylpiperidine-4-carboxamide hydrochloric acid compound (3.40 g, 11.8 mmol).

### [Step 2] Synthesis of 2-methyl-4-(2-(5-(((4-(4-fluorobenzyl)-4'-carboxamidyl)piperidine)methyl)furan-2-yl)phenyl)isoindoline-1-one

A 2-methyl-4-(2-(5-(((4-(4-fluorobenzyl)-4'-carboxamidyl)piperidine)methyl)furan-2-yl)phenyl)isoindoline-1-one compound was synthesized in the same manner as Example 1 using 4-(4-fluorobenzyl)-N-methylpiperidine-4-carboxamide hydrochloric acid prepared in Step 1 and 5-(2-(2-methyl-1-oxoisoindoline-4-yl)phenyl)furan-2-carboaldehyde prepared in Preparation Example 1-2).
¹H-NMR (400MHz, DMSO-d₆) δ 8.05 (d, 1H), 7.96(d, 2H), 7.94(d, 1H), 7.60(m, 2H), 7.51(t, 1H), 7.18(d, 2H), 7.16(d, 2H), 6.29(d, 1H), 4.22(brs, 2H), 3.76(s, 2H), 3.27(s, 3H), 2.80(d, 3H), 2.71(s, 2H), 2.51-2.45(m, 4H), 1.80-1.67(m, 4H)
MS(ESI+) m/z 552 (M+H)⁺

### Example 22: Synthesis of 2-methyl-4-(2-(5-((4-(4-fluorophenyl)-2λ², 8-diazaspiro[4,5]decan-1-one)methyl)furan-2-yl)phenyl)isoindoline-1-one

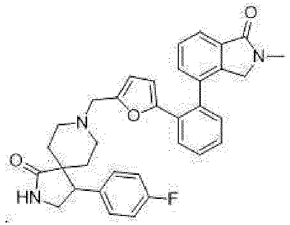

¹H-NMR (400MHz, DMSO-d₆) δ 8. 05 (d, 1H), 7.96(d, 2H), 7.94(d, 1H), 7.60(m, 2H), 7.50(t, 1H), 7.18(d, 2H), 7.16(d, 2H), 6.29(d, 1H), 4.22(brs, 2H), 3.81(m, 2H), 3.76(s, 2H), 3.27(s, 3H), 3.18(d, 2H), 2.71(s, 2H), 2.51-2.45(m, 4H), 1.80-1.67(m, 4H)
MS(ESI+) m/z 549 (M+H)⁺

### Example 23: Synthesis of 2-methyl-4-(2-(5-(((4-(4-fluorophenyl)-2-methyl)-2λ², 8-diazaspiro[4,5]decan-1-one)methyl)furan-2-yl)phenyl)isoindoline-1-one

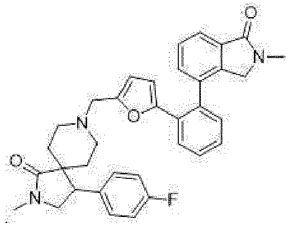

¹H-NMR (400MHz, DMSO-d₆) δ 8.05 (d, 1H), 7.98 (d, 2H), 7.94(d, 1H), 7.60(m, 2H), 7.50(t, 1H), 7.18(d, 2H), 7.16(d, 2H), 6.29(d, 1H), 4.22(brs, 2H), 3.81(m, 2H), 3.76(s, 2H), 3.27(s, 3H), 3.19(d, 2H), 2.94(s, 3H), 2.71(s, 2H), 2.51-2.45(m, 4H), 1.80-1.67(m, 4H)
MS(ESI+) m/z 564 (M+H)⁺

### Example 24: Synthesis of 2-methyl-4-(2-(5-((3-ethyl-3'-N-methyl-carboxamidyl)piperidine)methyl)furan-2-yl)phenyl)isoindoline-1-one

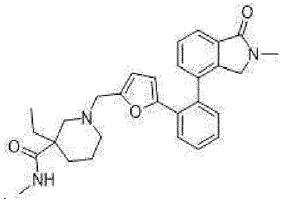

### [Step 1] Preparation of 3-ethyl-N-methylpiperidine-3-carboxamide hydrochloride

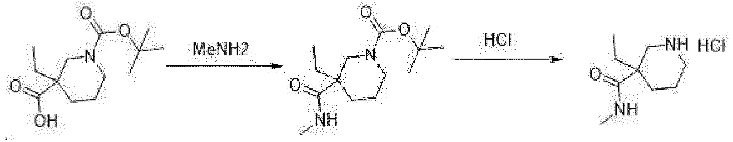

A 3-ethyl-N-methylpiperidine-3-carboxamide hydrochloride compound was prepared in the same manner as Step 1 of Example 23 using 1-(tert-butoxycarbonyl)-3-ethylpiperidine-3-carboxylic acid.

### [Step 2] Synthesis of 2-methyl-4-(2-(5-((3-ethyl-3'-N-methyl-carboxamidyl)piperidine)methyl)furan-2-yl)phenyl)isoindoline-1-one

A 2-methyl-4-(2-(5-((3-ethyl-3'-N-methyl-carboxamidyl)piperidine)methyl)furan-2-yl)phenyl)isoindoline-1-one compound was synthesized in the same manner as Example 1 using 3-ethyl-N-methylpiperidine-3-carboxamide hydrochloride prepared in Step 1 and 5-(2-(2-methyl-1-oxoisoindoline-4-yl)phenyl)furan-2-carboaldehyde prepared in Preparation Example 1-2).
¹H-NMR (400MHz, DMSO-d₆) δ 8.05 (d, 1H), 7.96(d, 2H), 7.94(d, 1H), 7.69(m, 1H), 7.60(m, 2H), 7.50(t, 1H), 6.29(d, 1H), 4.22(brs, 2H), 3.47(s, 2H), 3.27(s, 3H), 2.80(d, 3H), 2.48-2.40(m, 4H), 1.53-1.43(m,4H), 1.49(m, 2H), 0.89(t, 3H),
MS(ESI+) m/z 472 (M+H)⁺

### Example 25: Synthesis of 2-methyl-4-(2-(5-(((4-hydroxy)3-methyl-3'-N-methyl-carboxamidyl)piperidine)methyl)furan-2-yl)phenyl)isoindoline-1-one

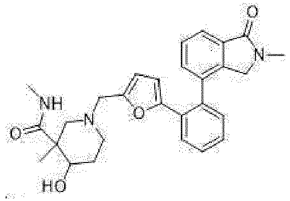

¹H-NMR (400MHz, DMSO-d₆) δ 8.05 (d, 1H), 7.96 (d, 2H), 7.94(d, 1H), 7.69(m, 1H), 7.60(m, 2H), 7.48(t, 1H), 6.77(d, 1H), 6.29(d, 1H), 4.22(brs, 2H), 3.80(m, 1H), 3.47(s, 2H), 3.27(s, 3H), 2.80(d, 3H), 2.65-2.59(m, 2H), 2.51-2.41(m, 2H), 1.77-1.52(m, 2H), 1.32(s, 3H)
MS(ESI+) m/z 474 (M+H)⁺

### Example 26: Synthesis of 2-methyl-4-(2-(5-((N-methyl-2-(3-oxopiperazine-2yl)acetamidyl)methyl)furan-2-yl)phenyl)isoindoline-1-one

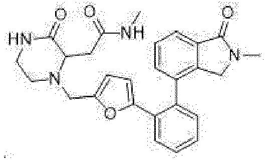

¹H-NMR (400MHz, DMSO-d₆) δ 8.05 (d, 1H), 7.96 (d, 2H), 7.94(d, 1H), 7.69(m, 1H), 7.60(m, 2H), 7.50(d, 1H), 7.52(t, 1H), 6.97(d, 1H), 6.29(d, 1H), 4.22(brs, 2H), 3.87(d, 1H), 3.47(s, 2H), 3.31(m, 2H), 3.27(s, 3H), 2.92-2.87(m, 2H), 2.80(s, 3H), 2.65(d, 2H),
MS(ESI+) m/z 473 (M+H)⁺

### Example 27: Synthesis of 2-methyl-4-(2-(5-((3-benzyl-N-methylpiperidine-3-carboxamidyl)methyl)furan-2-yl)phenyl)isoindoline-1-one

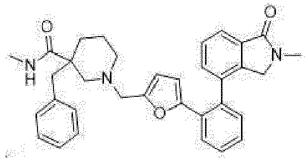

3-benzyl-N-methylpiperidine-3-carboxamide (1.0 g, 4.30 mmol) was dissolved in acetonitrile (50 ml) and added with potassium carbonate (891 mg, 6.45 mmol) and potassium iodide (71.38 mg, 0.43 mmol). The reaction solution was added with 4-(2-(5-(chloromethyl)furan-2-yl)phenyl)-2-methylisoindoline-1-one (1.45 g, 4.30 mmol) synthesized in Preparation Example 2-2) and stirred at room temperature for 7 hours. When the reaction was completed, the mixture was extracted by adding dichloromethane (150 ml) and water (150 ml), and the separated organic layer was concentrated under reduced pressure using anhydrous magnesium sulfate. The residue was separated and purified using silica gel column chromatography to obtain a 2-methyl-4-(2-(5-((3-benzyl-N-methylpiperidine-3-carboxamidyl)methyl)furan-2-yl)phenyl)isoindoline-1-one compound.
¹H-NMR (400MHz, DMSO-d₆) δ 8.05 (d, 1H), 7.96(d, 2H), 7.94(d, 1H), 7.69(m, 1H), 7.60(m, 2H), 7.50(m, 1H), 7.23(m, 2H), 7.21(d, 2H), 7.19(d, 1H), 4.22(brs, 2H), 3.47(s, 2H), 3.27(s, 3H), 2.84(s, 2H), 2.80(d, 3H), 2.65(s, 2H), 2.48(d, 2H), 1.80-1.72(m, 2H), 1.53-1.50(m, 2H)
MS(ESI+) m/z 534 (M+H)⁺

### Example 28: Synthesis of 2-methyl-4-(2-(5-((3-benzyl-N-methylpiperidine-3-carboxamidyl)methyl)furan-2-yl)phenyl)isoindoline-1-one

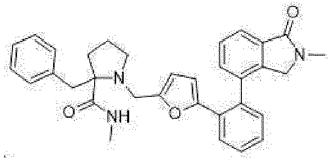

A 2-methyl-4-(2-(5-((3-benzyl-N-methylpiperidine-3-carboxamidyl)methyl)furan-2-yl)phenyl)isoindoline-1-one compound was obtained in the same manner as Example 27 using 2-benzyl-N-methylpyrrolidine-2-carboxamide.
¹H-NMR (400MHz, DMSO-d₆) δ 8.05 (d, 1H), 7.96(d, 2H), 7.94(d, 1H), 7.69(m, 1H), 7.60(m, 2H), 7.50(m, 1H), 7.23(m, 2H), 7.21(d, 2H), 7.19(d, 1H), 4.22(brs, 2H), 3.47(s, 2H), 3.27(s, 3H), 2.96(s, 2H), 2.82(d, 3H), 2.40(d, 2H), 1.90(d, 2H), 1.64(m, 2H)
MS(ESI+) m/z 521 (M+H)⁺

### Example 29: Synthesis of 2-methyl-4-(2-(5-(((4-benzyl-4'-carboxamidyl)piperidine)methyl)furan-2-yl)phenyl)isoindoline-1-one

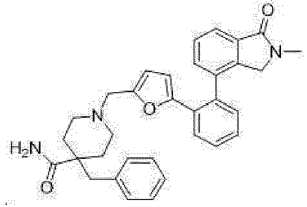

A 2-methyl-4-(2-(5-(((4-benzyl-4'-carboxamidyl)piperidine)methyl)furan-2-yl)phenyl)isoindoline-1-one compound was obtained in the same manner as Example 27 using 4-(benzyl)-4-piperidine-carboxamide.
¹H-NMR (400MHz, DMSO-d₆) δ 8.05 (d, 1H), 7.96(d, 2H), 7.94(d, 1H), 7.70(t, 1H), 7.65(d, 1H), 7.61(t, 1H), 7.50(t, 1H), 7.32(d, 1H), 7.21(d, 2H), 7.23(m, 2H), 7.19(m, 1H), 7.12(brs, 2H), 6.29(d, 1H), 4.22(brs, 2H), 3.76(s, 2H), 3.27(s, 3H), 2.71(s, 2H), 2.45(m, 4H), 1.80-1.67(m, 4H)
MS(ESI+) m/z 521 (M+H)⁺

### Example 30: Synthesis of 2-methyl-4-(2-(5-((3-benzyl-N-methylpiperidine-3-carboxamidyl)methyl)furan-2-yl)phenyl)isoindoline-1-one

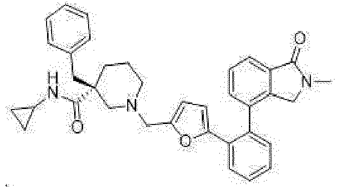

A 2-methyl-4-(2-(5-((3-benzyl-N-methylpiperidine-3-carboxamidyl)methyl)furan-2-yl)phenyl)isoindoline-1-one compound was obtained in the same manner as Example 27 using (3S)-N-cyclopropyl-3-(benzyl)-3-piperidine carboxamide.
¹H-NMR (400MHz, DMSO-d₆) δ 8.20(brs, 1H), 8.05 (d, 1H), 7.96(d, 2H), 7.94(d, 1H), 7.69(m, 1H), 7.60(m, 2H), 7.50(m, 1H), 7.23(m, 2H), 7.21(d, 2H), 7.19(d, 1H), 4.22(brs, 2H), 3.47(s, 2H), 3.27(s, 3H), 2.84(s, 2H), 2.69(m, 1H), 2.65(s, 2H), 2.48(d, 2H), 1.80-1.72(m, 2H), 1.53-1.50(m, 2H), 0.82(m, 2H), 0.57(m,2H)
MS(ESI+) m/z 560 (M+H)⁺

### Example 31: Synthesis of 2-methyl-4-(2-(5-(((4-pyridinylmethyl-4'-carboxamidyl)piperidine)methyl)furan-2-yl)phenyl)isoindoline-1-one

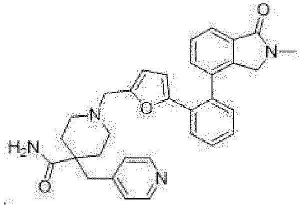

A 2-methyl-4-(2-(5-(((4-pyridinylmethyl-4'-carboxamidyl)piperidine)methyl)furan-2-yl)phenyl)isoindoline-1-one compound was obtained in the same manner as Example 27 using 4-(4-pyridinylmethyl)-4-piperidine-carboxamide.
¹H-NMR (400MHz, DMSO-d₆) δ 8.55 (d, 2H), 8.05 (d, 1H), 7.96(d, 2H), 7.94(d, 1H), 7.70(t, 1H), 7.69(d, 1H), 7.23(d, 2H), 7.10(brs, 2H), 6.29(d, 1H), 4.22(brs, 2H), 3.76(s, 2H), 3.27(s, 3H), 2.71(s, 2H), 2.45(m, 4H), 1.80-1.67(m, 4H)
MS(ESI+) m/z 521 (M+H)⁺

### Example 32: Synthesis of 2-methyl-4-(2-(5-(((4-(4-fluorobenzyl)-4'-carboxamidyl)piperidine)carbonyl)furan-2-yl)phenyl)isoindoline-1-one

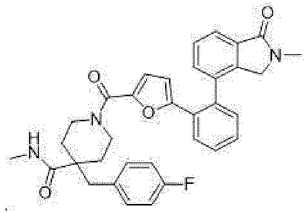

5-(2-(2-methyl-1-oxoisoindoline-4-yl)phenyl)furan-2-carboaldehyde (1.0 g, 3.15 mmol) synthesized in Preparation Example 1 was dissolved in acetonitrile (50 ml) and added with cobalt (II) iodide (24.6 mg, 0.078 mmol), calcium carbonate (346 mg, 3.47 mmol), tert-butyl hydroperoxide (70% in H₂O, 1.51 mL, 11.0 mmol), and 4-(4-fluorobenzyl)-N-methylpiperidine-4-carboxamide hydrochloric acid (3.47 mmol) prepared in Step 1 of Example 20 under a nitrogen stream, and stirred. The reaction solution was stirred at 70°C for 24 hours. When the reaction was completed, the mixture was cooled to room temperature, added with a saturated ammonium chloride aqueous solution (30 ml), and then stirred. The mixture was added with water and extracted with ethyl acetate (200 ml, twice). The extracted solution was dried with anhydrous magnesium sulfate and concentrated under reduced pressure to obtain a residue. The residue was separated by silica gel column chromatography to obtain a 2-methyl-4-(2-(5-(((4-(4-fluorobenzyl)-4'-carboxamidyl)piperidine)carbonyl)furan-2-yl)phenyl)isoindoline-1-one compound.
¹H-NMR (400MHz, DMSO-d₆) δ 8.06(d, 1H), 7.96(d, 2H), 7.94(d, 1H), 7.60(m, 2H), 7.50(t, 1H), 7.21(d, 1H), 7.18(d, 2H), 7.13(d, 2H), 4.22(brs, 2H), 3.27(s, 3H), 2.80(d, 3H), 2.71(s, 2H), 2.51-2.45(m, 4H), 1.80-1.67(m, 4H)
MS(ESI+) m/z 566 (M+H)⁺

### Example 33: Synthesis of 2-methyl-4-(2-(5-((methylamino)carbonyl)furan-2-yl)phenyl)isoindoline-1-one

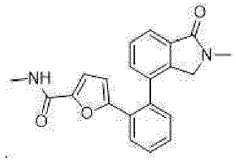

A 2-methyl-4-(2-(5-((methylamino)carbonyl)furan-2-yl)phenyl)isoindoline-1-one compound was obtained in the same manner as Example 32 using methylamine hydrochloride.
¹H-NMR (400MHz, DMSO-d₆) δ 8.04 (d, 1H), 7.97 (d, 2H), 7.94(d, 1H), 7.70(t, 1H), 7.66(d, 1H), 7.61(t, 1H), 7.56(d, 1H), 7.21(d, 1H), 7.13(d, 1H), 4.22(brs, 2H), 3.27(s, 3H), 2.85(d, 3H)
MS(ESI+) m/z 347 (M+H)⁺

### Example 34: Synthesis of 2-methyl-4-(2-(5-(((4-hydroxy)3-methyl-3'-N-methyl-carboxamidyl)piperidine)carbonyl)furan-2-yl)phenyl)isoindoline-1-one

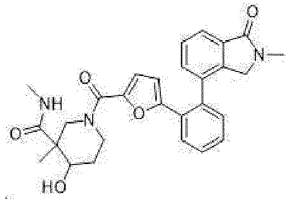

A 2-methyl-4-(2-(5-(((4-hydroxy)3-methyl-3'-N-methyl-carboxamidyl)piperidine)carbonyl)furan-2-yl)phenyl)isoindoline-1-one compound was obtained in the same manner as Example 32 using 4-hydroxy3-methyl-3'-N-methyl-carboxamidyl-piperidine.
¹H-NMR (400MHz, DMSO-d₆) δ 8.05 (d, 1H), 7.96(d, 2H), 7.94(d, 1H), 7.69(m, 1H), 7.60(m, 2H), 7.48(t, 1H), 7.21(d, 1H), 7.13(d, 1H), 6.77(d, 1H), 4.22(brs, 2H), 3.80(m, 1H), 3.27(s, 3H), 2.80(d, 3H), 2.65-2.59(m, 2H), 3.59-3.49(m, 2H), 1.93-1.68(m, 2H), 1.32(s, 3H)
MS(ESI+) m/z 488 (M+H)⁺

### Example 35: Synthesis of 2-methyl-4-(2-(5-((N-methyl-2-(3-oxopiperazine-2yl)acetamidyl)carbonyl)furan-2-yl)phenyl)isoindoline-1-one

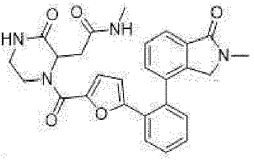

A 2-methyl-4-(2-(5-((N-methyl-2-(3-oxopiperazine-2yl)acetamidyl)carbonyl)furan-2-yl)phenyl)isoindoline-1-one compound was obtained in the same manner as Example 32 using N-methyl-2-(3-oxopiperazine-2yl)acetamidyl.
¹H-NMR (400MHz, DMSO-d₆) δ 8.05 (d, 1H), 7.96(d, 2H), 7.94(d, 1H), 7.69(m, 1H), 7.60(m, 2H), 7.50(d, 1H), 7.52(t, 1H), 7.21(d, 1H), 7.13(d, 1H), 4.84(m, 1H), 4.22(brs, 2H), 3.47(s, 2H), 3.31-3.28(m, 2H), 3.27(s, 3H), 3.01-2.80(m, 2H), 2.80(d, 3H)
MS(ESI+) m/z 487 (M+H)⁺

### Example 36: Synthesis of 2-methyl-4-(2-(5-((3-ethyl-3'-N-methyl-carboxamidyl)piperidine)carbonyl)furan-2-yl)phenyl)isoindoline-1-one

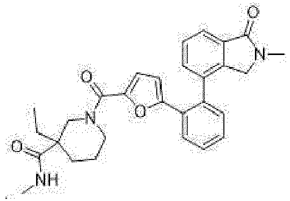

A 2-methyl-4-(2-(5-((3-ethyl-3'-N-methyl-carboxamidyl)piperidine)carbonyl)furan-2-yl)phenyl)isoindoline-1-one compound was obtained in the same manner as Example 32 using 3-ethyl-3'-N-methyl-carboxamidyl)piperidine.
¹H-NMR (400MHz, DMSO-d₆) δ 8.05 (d, 1H), 7.96(d, 2H), 7.94(d, 1H), 7.69(m, 1H), 7.60(m, 2H), 7.50(t, 1H), 7.13(d, 1H), 4.22(brs, 2H), 3.56-3.52(m, 4H), 3.27(s, 3H), 2.80(d, 3H), 1.68-1.58(m,4H), 1.48(m, 2H), 0.89(t, 3H)
MS(ESI+) m/z 486 (M+H)⁺

### Example 37: Synthesis of 2-methyl-4-(2-(5-((((4,4-difluoropiperidine-1-yl)methoxy)-3-carboxamidyl)methyl)furan-2-yl)phenyl)isoindoline-1-one

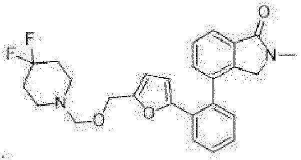

4,4-difluoropiperidine-1-yl)methanol (0.53 g, 3.50 mmol) was dissolved in dimethylformamide (20 ml) and slowly added dropwise with sodium hydride (60%, 154 mg, 3.85 mmol) at 0°C. The mixture was stirred for 1 hour at the same temperature, slowly added dropwise with 4-(2-(5-(chloromethyl)furan-2-yl)phenyl)-2-methylisoindoline-1-one (1.29 g, 3.85 mmol) synthesized in Preparation Example 2-2) and then stirred at room temperature for 4 hours. When the reaction was completed, the mixture was extracted by adding ethyl acetate (150 ml) and water (150 ml, twice), and the separated organic layer was concentrated under reduced pressure using anhydrous magnesium sulfate. The residue was separated and purified using silica gel column chromatography to obtain a 2-methyl-4-(2-(5-((((4,4-difluoropiperidine-1-yl)methoxy)-3-carboxamidyl)methyl)furan-2-yl)phenyl)isoindoline-1-one compound.
¹H-NMR (400MHz, DMSO-d₆) δ 8.05 (d, 1H), 7.96(d, 2H), 7.94(d, 1H), 7.69(m, 1H), 7.60(m, 2H), 6.98(d, 1H), 6.59(d, 1H), 4.48(s, 2H), 4.44(s, 2H), 4.22(brs, 2H), 3.27(s, 3H), 2.45(m, 4H), 1.82(m, 4H)
MS(ESI+) m/z 453 (M+H)⁺

### Examples 38 to 42 below were synthesized in the same manner as in Example 1 using 3-methyl-5-(2-(2-methyl-1-oxoisoindoline-4-yl)phenyl)furan-2-carboaldehyde obtained in Preparation Example 3, or prepared using an appropriate reactant in consideration of the structures of the compounds to be prepared.

### Example 38: Synthesis of 2-methyl-4-(2-(3-methyl-5-((methylamino)methyl)furan-2-yl)phenyl)isoindoline-1-one

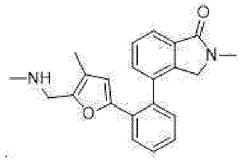

A 2-methyl-4-(2-(3-methyl-5-((methylamino)methyl)furan-2-yl)phenyl)isoindoline-1-one compound was obtained in the same manner as Example 1 using 3-methyl-5-(2-(2-methyl-1-oxoisoindoline-4-yl)phenyl)furan-2-carboaldehyde synthesized in Preparation Example 3 and methylamine hydrochloride.
¹H-NMR (400MHz, DMSO-d₆) δ 8.05 (d, 1H), 7.97 (d, 2H), 7.94(d, 1H), 7.70(t, 1H), 7.66(d, 1H), 7.61(t, 1H), 6.33(d, 1H), 4.16(m, 1H), 4.22(brs, 2H), 3.65(d, 2H), 3.27(s, 3H), 3.25(d, 3H), 2.01(s, 3H)
MS(ESI+) m/z 347 (M+H)⁺

### Example 39: Synthesis of 2-methyl-4-(2-(5-((3-methyl(4-(4-fluorobenzyl)-4'-carboxamidyl)piperidine)methyl)furan-2-yl)phenyl)isoindoline-1-one

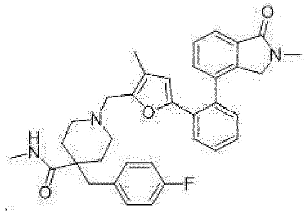

¹H-NMR (400MHz, DMSO-d₆) δ 8.05 (d, 1H), 7.96(d, 2H), 7.94(d, 1H), 7.60(m, 2H), 7.51(t, 1H), 7.18(d, 2H), 6.33(d, 1H), 4.22(brs, 2H), 3.76(s, 2H), 3.27(s, 3H), 2.80(d, 3H), 2.71(s, 2H), 2.51-2.45(m, 4H), 2.01(s, 3H), 1.80-1.67(m, 4H)
MS(ESI+) m/z 566 (M+H)⁺

### Example 40: Synthesis of 2-methyl-4-(2-(5-(3-methyl(3-ethyl-3'-N-methyl-carboxamidyl)piperidine)methyl)furan-2-yl)phenyl)isoindoline-1-one

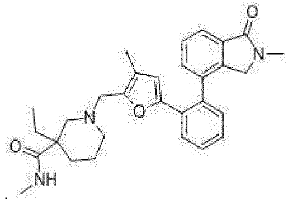

¹H-NMR (400MHz, DMSO-d₆) δ 8.05 (d, 1H), 7.96(d, 2H), 7.94(d, 1H), 7.69(m, 1H), 7.60(m, 2H), 7.50(t, 1H), 6.29(d, 1H), 4.22(brs, 2H), 3.47(s, 2H), 3.27(s, 3H), 2.80(d, 3H), 2.48-2.40(m, 4H), 2.01(s, 3H), 1.53-1.43(m,4H), 1.49(m, 2H), 0.89(t, 3H),
MS(ESI+) m/z 486 (M+H)⁺

### Example 41: Synthesis of 2-methyl-4-(2-(5-((3-methyl(4-hydroxy)3-methyl-3'-N-methyl-carboxamidyl)piperidine)methyl)furan-2-yl)phenyl)isoindoline-1-one

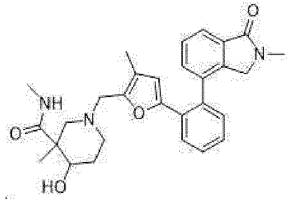

¹H-NMR (400MHz, DMSO-d₆) δ 8.05 (d, 1H), 7.96(d, 2H), 7.94(d, 1H), 7.69(m, 1H), 7.60(m, 2H), 7.48(t, 1H), 6.33(d, 1H), 4.22(brs, 2H), 3.80(m, 1H), 3.47(s, 2H), 3.27(s, 3H), 2.80(d, 3H), 2.65-2.59(m, 2H), 2.51-2.41(m, 2H), 2.01(s, 3H), 1.77-1.52(m, 2H), 1.32(s, 3H)
MS(ESI+) m/z 488 (M+H)⁺

### Example 42: Synthesis of 2-methyl-4-(2-(5-(3-methyl(N-methyl-2-(3-oxopiperazine-2yl)acetamidyl)methyl)furan-2-yl)phenyl)isoindoline-1-one

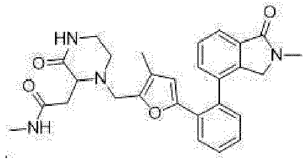

¹H-NMR (400MHz, DMSO-d₆) δ 8.05 (d, 1H), 7.96(d, 2H), 7.94(d, 1H), 7.69(m, 1H), 7.60(m, 2H), 7.50(d, 1H), 7.52(t, 1H), 6.33(d, 1H), 4.22(brs, 2H), 3.87(d, 1H), 3.47(s, 2H), 3.31(m, 2H), 3.27(s, 3H), 2.92-2.87(m, 2H), 2.80(s, 3H), 2.65(d, 2H), 2.01(s, 3H)
MS(ESI+) m/z 487 (M+H)⁺

### [Comparative Example]

Compounds of Comparative Examples 1 to 3 shown in Table 3 below were prepared and a BET inhibitory effect experiment was performed.

**[Table 3]**

| Com. Ex. | Structural Formula |
|---|---|
| Com. Ex. 1 | 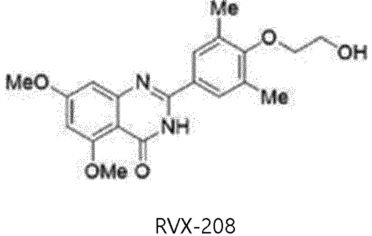 |
| Com. Ex. 2 | 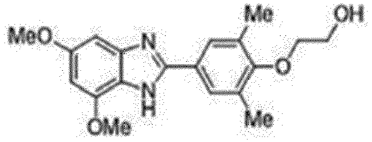 |
| Com. Ex. 3 | 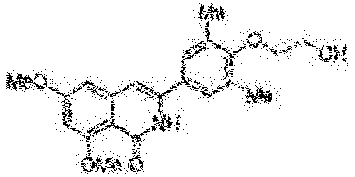 |

### [Experimental Example 1]

### Evaluation of binding inhibitory effect on BRD proteins

In order to evaluate the ability of a novel compound according to the present disclosure to inhibit the interaction between a bromodomain of BRD2 (BD1) and BRD3 (BD1), one of the BRD protein family, and a tetraacetylated histone H4 peptide, the following experiment was performed.

### 1-1. Binding inhibitory effect on BRD2 protein

The binding inhibitory effects of the compounds of the present disclosure and the compounds of Comparative Examples on the BRD2 protein among BRD proteins were experimented as follows.

Compounds were diluted in 1 : 5 serial dilutions in an assay buffer from a 10 mM stock in DMSO (100 µM starting concentration) in a white OptiPlate-384 (PerkinElmer). A mixture consisting of 100 nM GST-BRD2 (BD1) and 100 nM biotinylated acetyl-histone H4 (Lys5, 8, 12, 16) peptides was prepared in an assay buffer (50 mM HEPES pH 7.4; 25 mM NaCl; 0,05% Tween 20; 0.1% bovine serum albumin (BSA); 10 mM dithiothreitol (DTT)). 6 µl of the mixture was added to the dilution, and then added with 6 µl of premixed AlphaLISA Glutathione Acceptor Beads and AlphaScreen Streptavidin Donor Beads from PerkinElmer in the assay buffer at a concentration of 10 µg/ml, respectively, and samples were shaking-cultured at 300 rpm for 30 minutes at room temperature in the dark. Thereafter, a signal was measured with a PerkinElmer Envision HTS Multilabel Reader using a PerkinElmer AlphaScreen protocol. Each plate contained a negative control group in which a biotinylated acetyl-histone H4 peptide and GST-BRD2 (BD1) were removed and replaced with an assay buffer.

When using the software GraphPad Prism for calculation, the negative control value was input as a low reference value. Additionally, a positive control group (probe molecule I-BET762 with a protein/peptide mixture) was pipetted. The determination of the IC50 value was performed using the GraphPad Prism 3.03 software (or an updated version thereof), and the results were shown in Table 4 below.

**[Table 4]**

| Compound | BRD2 (BD1) |
|---|---|
| Com. Ex. 1 | 32,005 |
| Com. Ex. 2 | 65,151 |
| Com. Ex. 3 | 29,758 |
| Compound 1 | 16,894 |
| Compound 2 | 16,762 |
| Compound 3 | 14,124 |
| Compound 4 | 17,445 |
| Compound 5 | 12,382 |
| Compound 6 | 15,818 |
| Compound 7 | 18,187 |
| Compound 8 | 16, 578 |
| Compound 9 | 19,690 |
| Compound 10 | 11,380 |
| Compound 11 | 16,624 |
| Compound 12 | 14,488 |
| Compound 13 | 7,653 |
| Compound 14 | 13,338 |
| Compound 15 | 15,793 |
| Compound 16 | 15,841 |
| Compound 17 | 13,973 |
| Compound 18 | 6,508 |
| Compound 19 | 13,861 |
| Compound 20 | 16,721 |
| Compound 21 | 13,853 |
| Compound 22 | 13,707 |
| Compound 23 | 15,297 |
| Compound 24 | 15,185 |
| Compound 25 | 7,682 |
| Compound 26 | 12,957 |
| Compound 27 | 14,107 |
| Compound 28 | 10,945 |
| Compound 29 | 14,598 |
| Compound 30 | 5,940 |
| Compound 31 | 14,981 |
| Compound 32 | 16,848 |
| Compound 33 | 11,108 |
| Compound 34 | 15,699 |
| Compound 35 | 12,302 |
| Compound 36 | 14,462 |
| Compound 37 | 9,500 |
| Compound 38 | 12,484 |
| Compound 39 | 8,669 |
| Compound 40 | 10,552 |
| Compound 41 | 15,462 |
| Compound 42 | 10,923 |

As shown in Table 4 above, the compounds of the present disclosure showed lower IC50 values of BRD2 (BD1) than the compounds of Comparative Examples. Therefore, it was confirmed that the compounds of the present disclosure had better inhibitory activity on the BRD2 (BD1) protein than existing BRD inhibitors.

### 1-2. Binding inhibitory effect on BRD3 protein

An experiment was performed to confirm the binding inhibitory effects of the compounds of the present disclosure and the compounds of Comparative Examples on the BRD3 (BD1) protein in the same manner as Experimental Example 1-1. The results were shown in Table 5 below.

**[Table 5]**

| Compound | BRD3 (BD1) |
|---|---|
| Com. Ex. 1 | 23,398 |
| Com. Ex. 2 | >50,000 |
| Com. Ex. 3 | 45,280 |
| Compound 1 | 1,394 |
| Compound 2 | 1,263 |
| Compound 3 | 4,510 |
| Compound 4 | 4,426 |
| Compound 5 | 2,356 |
| Compound 6 | 2,605 |
| Compound 7 | 4,354 |
| Compound 8 | 2,204 |
| Compound 9 | 3,855 |
| Compound 10 | 3,887 |
| Compound 11 | 3,707 |
| Compound 12 | 2,987 |
| Compound 13 | 3,645 |
| Compound 14 | 2,401 |
| Compound 15 | 2,555 |
| Compound 16 | 1,754 |
| Compound 17 | 3,612 |
| Compound 18 | 2,670 |
| Compound 19 | 1,404 |
| Compound 20 | 3,718 |
| Compound 21 | 1,163 |
| Compound 22 | 1,194 |
| Compound 23 | 1,299 |
| Compound 24 | 1,666 |
| Compound 25 | 1,295 |
| Compound 26 | 1,380 |
| Compound 27 | 2,541 |
| Compound 28 | 4,357 |
| Compound 29 | 4,862 |
| Compound 30 | 2,571 |
| Compound 31 | 2,785 |
| Compound 32 | 3,987 |
| Compound 33 | 2,393 |
| Compound 34 | 4,678 |
| Compound 35 | 1,221 |
| Compound 36 | 4,257 |
| Compound 37 | 4,763 |
| Compound 38 | 1,118 |
| Compound 39 | 4,156 |
| Compound 40 | 3,956 |
| Compound 41 | 2,136 |
| Compound 42 | 3,773 |

As shown in Table 5 above, it can be seen that the compounds of the present disclosure have much lower IC50 values of BRD3 (BD1) than the compounds of Comparative Examples. Therefore, it was confirmed that the compounds of the present disclosure had a better BRD3 (BD1) protein inhibitory effect than the existing BRD inhibitor RVX-208[ 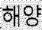 1].

### [Experimental Example 2]

### Evaluation of retinal degeneration inhibitory effect in retinal degeneration mouse model

In order to fabricate a retinal degeneration model, 7-week-old male albino BALB/c mice were used. Three mice were randomly assigned to each test group, and light and dark were maintained at 12-hour intervals. Each mouse was dark-adapted for 24 hours and then the pupils were dilated with 0.5% tropicamide and 0.5% phenylephrine hydrochloride eye drops (Santen, Osaka, Japan) 30 minutes before blue LED exposure. Unanesthetized mice were exposed to 2,000 lux of a blue LED (460 ± 10 nm) for 2 hours in a cage with reflective interiors. The light intensity was measured using an LED photometer (model TM-201L, TENMARS Electronics, Taipei, Taiwan). After exposure to blue LED, the mice were kept in the dark for 24 hours and then resumed on a 12-hour light/dark cycle for 3 days.

In a test substance control test, mice with retinal degeneration were divided into a normal saline-administered group and a compound-administered group (24 nM) of the present disclosure, and 3 mice were randomly assigned to each test group. A positive control test was divided into a normal saline-administered group and an Eyela (25 mg/kg mouse body weight)-administered group (Comparative Example 4), and three mice were randomly assigned to each test group.

All test groups were administered as a single dose by intravitreal injection 1 hour after LED exposure.

### Electroretinogram (ERG)

Electroretinogram (ERG) recordings followed the experimental procedure presented by Kim *et al.* (Kim, G. H., Kim, H. I., Paik, S. S., Jung, S. W., Kang, S., and Kim, I. B. (2016). Functional and morphological evaluation of blue light-emitting diode-induced retinal degeneration in mice. Graefes Arch. Clin. Exp. Ophthalmol. 254, 705-716).

That is, mice were stored in a completely dark room for 16 hours before ERG recordings. All animals were anesthetized intraperitoneally with zolazepam (20 mg/kg) and xylazine (7.5 mg/kg). The cornea was coated with hydroxypropyl methylcellulose gel and covered with a gold ring contact electrode. Ground and reference electrodes were disposed subcutaneously on the tail and ears, respectively. Stimuli were short white flashes delivered through a Ganzfeld stimulator (UTAS-3000; LKC Technologies, Gaithersburg, MD, USA). The signal was amplified and filtered through a digital band-pass filter in the range of 5 to 300 Hz to generate a and b waves. Scotopic ERG, and rod-mediated responses were obtained at increasing light intensities of 0.025 and 3.96 cd/s • m². Photopic, cone-mediated responses were obtained after 5-min light adaptation to background light intensity. The recordings were obtained at the light intensity of 6.28 cd/s • m². Each recording was the average of three responses obtained within a 15-second stimulation interval. A-wave amplitude was measured from a baseline to the maximum a-wave peak, and b-wave amplitude was measured from the maximum a-wave peak to the maximum b-wave peak.

The ERG analysis results were shown in Table 6 below.

**[Table 6]**

| Compound | Wavelength enhanced multiple compared to normal saline-treated group | | |
|---|---|---|---|
| | a-wave | b-wave | Average value |
| Com. Ex. 4 | 1.25 | 1.20 | 1.23 |
| Compound 13 | 1.29 | 1.36 | 1.33 |
| Compound 18 | 2.05 | 2.03 | 2.04 |
| Compound 21 | 2.17 | 2.42 | 2.30 |
| Compound 25 | 1.52 | 1.01 | 1.27 |
| Compound 28 | 1.31 | 1.35 | 1.33 |
| Compound 30 | 2.25 | 1.24 | 1.75 |
| Compound 37 | 1.45 | 1.31 | 1.38 |
| Compound 39 | 1.21 | 1.51 | 1.36 |
| Compound 40 | 1.16 | 1.43 | 1.30 |
| Compound 42 | 1.25 | 1.38 | 1.32 |

As shown in Table 6 above, when the increased wavelength compared to a normal saline-treated group was expressed as an enhanced multiple, the group administered with the compound of the present disclosure was found to be excellent in an increase in wavelength compared to the group administered Eyela, which was commercially available as a macular degeneration therapeutic agent. Therefore, it was confirmed that the compound of the present disclosure exhibited a better retinal degeneration inhibitory effect than the existing macular degeneration therapeutic agent (Eylea).

### [Experimental Example 3]

### Evaluation of retinal degeneration inhibitory effect in retinal degeneration mouse model

After the mouse was subjected to general anesthesia, the eye was applied with anesthetic eye drops to be subjected to additional local anesthesia and additionally induce mydriasis. A macular degeneration animal model was prepared by destroying the Bruch's membrane by inducing laser burn according to macular degeneration induction conditions. Test substances (Compounds 1 to 42; 0.022 µg/eye), Comparative Example 4 as a control substance (aflibercept (Eylea); 20 µg/eye), and a negative control substance (excipient) were injected with a 36G needle through a scleral puncture immediately after macular degeneration induction, and then administered to both eyes at a volume of 1 uL/eye. On day 11 of macular degeneration induction, the mice were subjected to general anesthesia and then injected intraperitoneally with a fluorescent contrast agent. The mouse was placed on the sacrifice table, lubricating gel was dropped into the corresponding eye, and an OCT lens was brought into contact with the mouse's cornea. Image analysis for fluorescein fundus angiography (FFA) and OCT was performed using the Image-J program.

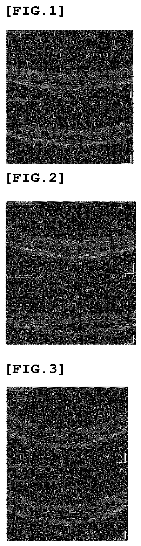

The analysis results from the OCT imaging were shown in Table 7 below and FIGS. 1 to 3.

**[Table 7]**

| Compound | Volume of lesion (µm³) | Lesion size compared to negative control group (%) |
|---|---|---|
| Com. Ex. 4 | 1,298,967 | 59.63 |
| Compound 13 | 1,256,002 | 57.66 |
| Compound 18 | 983,477 | 45.15 |
| Compound 21 | 888,502 | 40.79 |
| Compound 25 | 958, 694 | 44.01 |
| Compound 28 | 1,132,542 | 51.99 |
| Compound 30 | 957,608 | 43.96 |
| Compound 37 | 1,010,752 | 46.40 |
| Compound 39 | 1,265,636 | 58.10 |
| Compound 40 | 1,176,522 | 54.01 |
| Compound 42 | 1,151,561 | 52.86 |

As shown in Table 7 and FIGS. 1 to 3, as a result of analyzing changes in macular degeneration lesions, it was shown that in the group administered with the compound of the present disclosure, the size of macular degeneration lesions was statistically significantly decreased compared to the negative control group. In addition, it was shown that the CTF value was statistically significantly more decreased in the group administered with the compound of the present disclosure compared to the group administered with Eylea, a control substance.

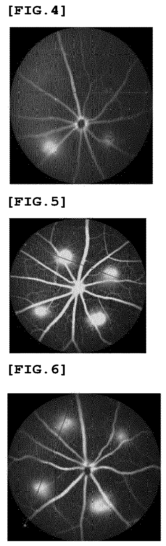

In addition, the analysis results from the FFA imaging were shown in Table 8 below and FIGS. 4 to 6.

**[Table 8]**

| Compound | CTF value (Pixels) | Lesion size compared to negative control group (%) |
|---|---|---|
| Com. Ex. 4 | 390,624 | 42.43 |
| Compound 13 | 321,816 | 34.96 |
| Compound 18 | 389,222 | 42.28 |
| Compound 21 | 251,239 | 27.29 |
| Compound 25 | 300,650 | 32.66 |
| Compound 28 | 325,936 | 35.40 |
| Compound 30 | 325,433 | 35.35 |
| Compound 37 | 320,028 | 34.76 |
| Compound 39 | 252,108 | 27.38 |
| Compound 40 | 362,456 | 39.37 |
| Compound 42 | 381,846 | 41.48 |

As shown in Table 8 and FIGS. 4 to 6, as a result of analyzing changes in macular degeneration lesions, it was shown that in the group administered with the compound of the present disclosure, the size of macular degeneration lesions was statistically significantly decreased compared to the negative control group. It was shown that in the group administered with the compound of the present disclosure, the volume of macular degeneration lesions was also reduced more than in the group administered with Eylea, a control substance.

Therefore, it was confirmed that the compound of the present disclosure exhibited a better macular degeneration inhibitory effect than the existing macular degeneration therapeutic agent (Eylea).

## Claims

1. A compound of Chemical Formula I below, a solvate, a stereoisomer or a pharmaceutically acceptable salt thereof: 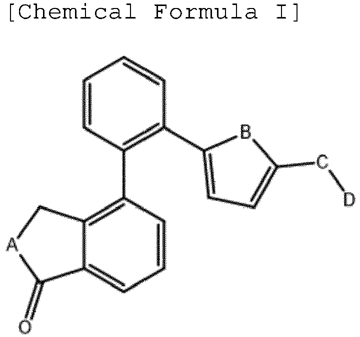 wherein,
A and B are each independently any one selected from the group consisting of oxygen, nitrogen, and sulfur, and when A or B is nitrogen, the hydrogen bonded to nitrogen is substituted with an alkyl group,
C is any one selected from the group consisting of hydrogen, C₁₋₆ alkyl, and carbonyl, and
D is hydrogen, C₁₋₆ alkyl, -NH₂, -NH(C₁₋₆ alkyl), -N(C₁₋₆ alkyl) (C₁₋₆ alkyl), -NH(C₁₋₆ cycloalkyl), cycloalkyl, heterocycloalkyl, aryl or heteroaryl.

2. The compound of Chemical Formula I, the solvate, the stereoisomer or the pharmaceutically acceptable salt thereof of claim 1, wherein a hydrogen bonding moiety of the -NH (C₁₋₆ alkyl), -N(C₁₋₆ alkyl) (C₁₋₆ alkyl), -NH(C₁₋₆ cycloalkyl), cycloalkyl, heterocycloalkyl, aryl, and heteroaryl may be substituted with at least one substituent selected from the group consisting of hydrogen, halogen, -OH, =O, -OC₁₋₆ alkyl, -C₁₋₆ alkyl, -C(=O)Ra, -C(=O)N(Ra)(Rb), -C₁₋₆ alkylC(=O)N(Ra)(Rb), -C₃H₄C(=O)N(Ra)(Rb), -C=CH, -C₃₋₆ cycloalkyl, -N(C₁₋₆ alkyl) (C₁₋₆ alkyl), -SO₂(C₁₋₆ alkyl), phenyl, phenyl in which at least one of hydrogen or carbon is substituted with halogen, methyl piperazinyl, pyrazoyl in which any one of hydrogens is substituted with C₁₋₆ alkyl, pyrrolidonyl in which any one of hydrogens is substituted with halogenated phenyl, methyl pyrrolidonyl in which any one of hydrogens is substituted with halogenated phenyl, benzyl, benzyl in which at least one of hydrogen or carbon is substituted with nitrogen, and benzyl in which at least one of hydrogen or carbon is substituted with halogen, and
the Ra and Rb are each independently H, C₁₋₆ alkyl, C₃₋₆ cycloalkyl, and benzyl.

3. The compound of Chemical Formula I, the solvate, the stereoisomer or the pharmaceutically acceptable salt thereof of claim 2, wherein the heterocycloalkyl of the D includes at least one selected from the group consisting of pyrrolidinyl, piperidinyl, piperazinyl, methyl piperazinyl, morpholinyl, and piperazinonyl.

4. The compound of Chemical Formula I, the solvate, the stereoisomer or the pharmaceutically acceptable salt thereof of claim 2, wherein the C is either methyl or carbonyl.

5. A compound of Chemical Formula II below, a solvate, a stereoisomer or a pharmaceutically acceptable salt thereof: 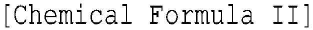 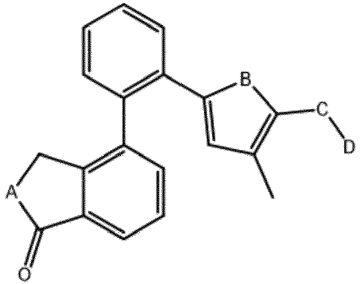 wherein,
A and B are each independently any one selected from the group consisting of oxygen, nitrogen, and sulfur, and when A or B is nitrogen, the hydrogen bonded to nitrogen is substituted with an alkyl group,
C is hydrogen or C₁₋₆ alkyl,
D is hydrogen, C₁₋₆ alkyl, -NH₂, -NH(C₁₋₆ alkyl), -N(C₁₋₆ alkyl)(C₁₋₆ alkyl), cycloalkyl, heterocycloalkyl, aryl or heteroaryl,
the cycloalkyl, heterocycloalkyl, aryl, and heteroaryl may be substituted with at least one substituent selected from the group consisting of hydrogen, -OH, =O, -C₁₋₆ alkyl, -C(=O)Ra, - C(=O)N(Ra)(Rb), -C₁₋₆ alkylC(=O)N(Ra)(Rb), and benzyl in which at least one of hydrogen or carbon is substituted with halogen,
the heterocycloalkyl includes at least one selected from the group consisting of pyrrolidinyl, piperidinyl, piperazinyl, methyl piperazinyl, morpholinyl, and piperazinonyl, and
the Ra and Rb are each independently H or C₁₋₆ alkyl.

6. The compound of Chemical Formula II, the solvate, the stereoisomer or the pharmaceutically acceptable salt thereof of claim 5, wherein the C is methyl.

7. The compound of Chemical Formula I, the solvate, the stereoisomer or the pharmaceutically acceptable salt thereof of claim 1, wherein the compound is selected from the group consisting of compounds represented by the following Chemical Formulas:
| | Chemical Formula |
|---|---|
| Compound 1 | 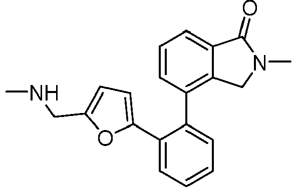 |
| Compound 2 | 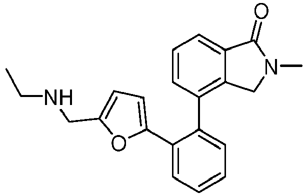 |
| Compound 3 | 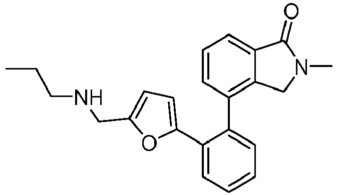 |
| Compound 4 | 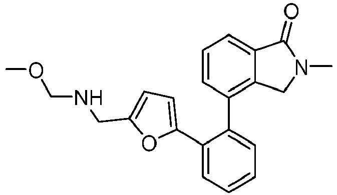 |
| Compound 5 | 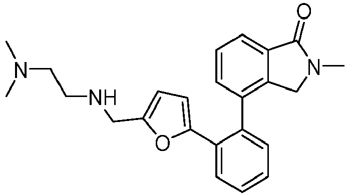 |
| Compound 6 | 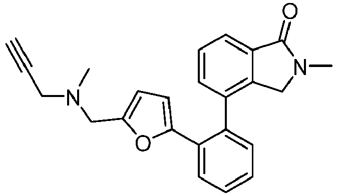 |
| Compound 7 | 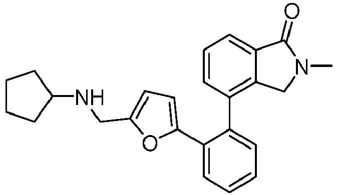 |
| Compound 8 | 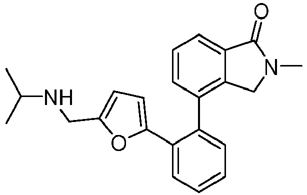 |
| Compound 9 | 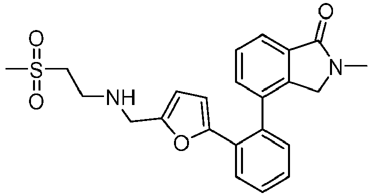 |
| Compound 10 | 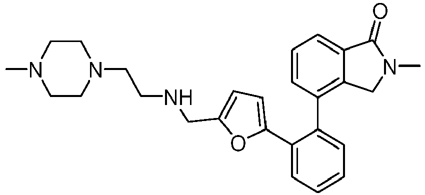 |
| Compound 11 | 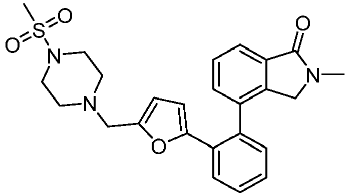 |
| Compound 12 | 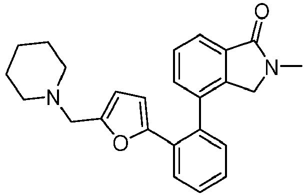 |
| Compound 13 | 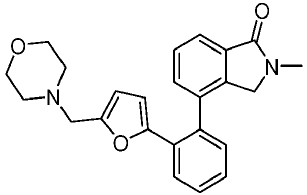 |
| Compound 14 | 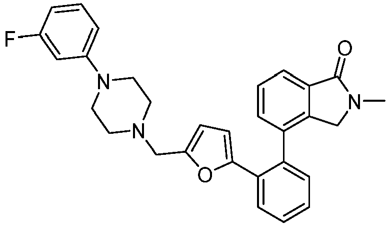 |
| Compound 15 | 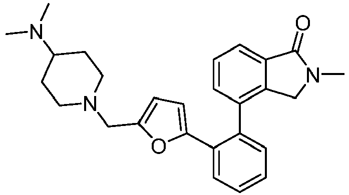 |
| Compound 16 | 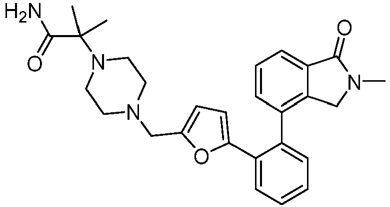 |
| Compound 17 | 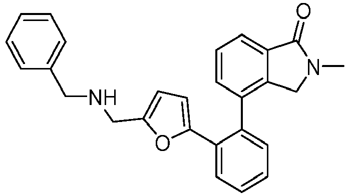 |
| Compound 18 | 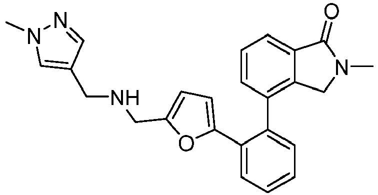 |
| Compound 19 | 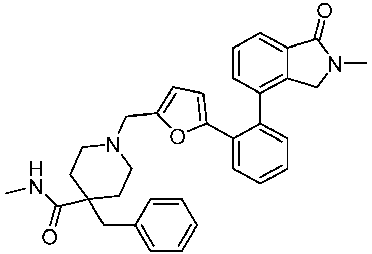 |
| Compound 20 | 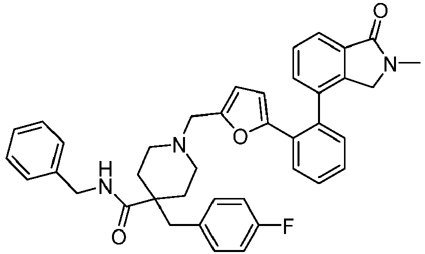 |
| Compound 21 | 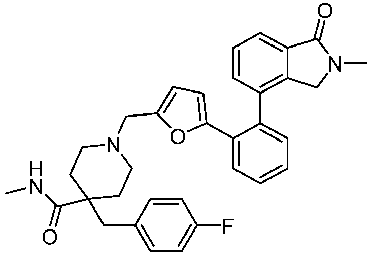 |
| Compound 22 | 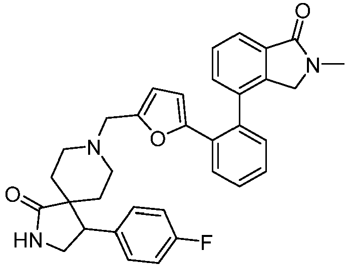 |
| Compound 23 | 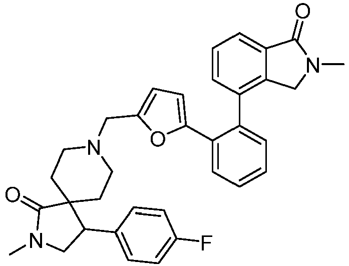 |
| Compound 24 | 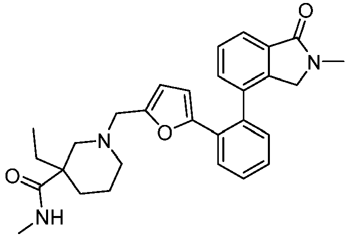 |
| Compound 25 | 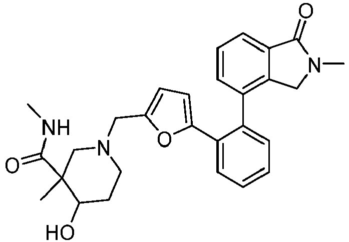 |
| Compound 26 | 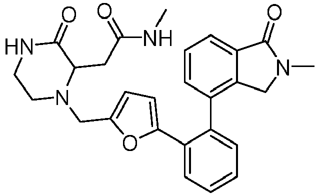 |
| Compound 27 | 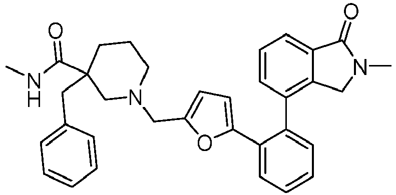 |
| Compound 28 | 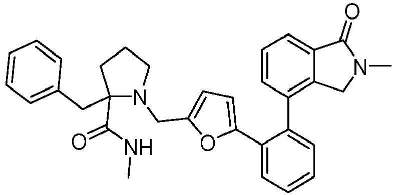 |
| Compound 29 | 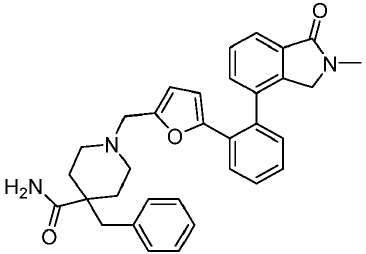 |
| Compound 30 | 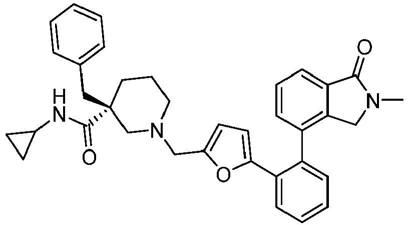 |
| Compound 31 | 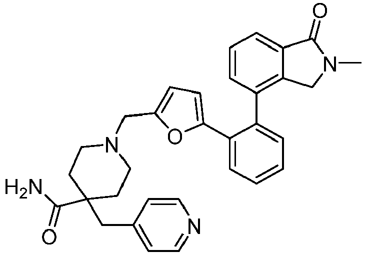 |
| Compound 32 | 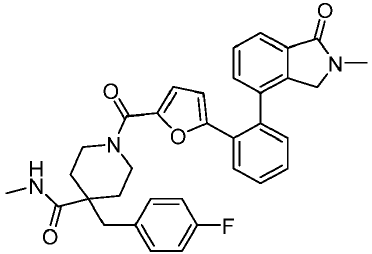 |
| Compound 33 | 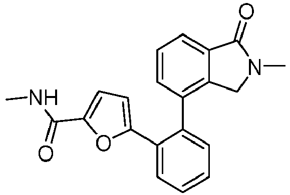 |
| Compound 34 | 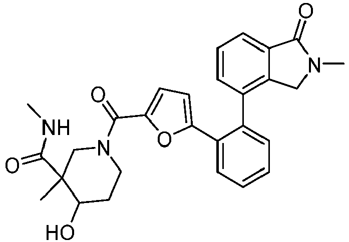 |
| Compound 35 | 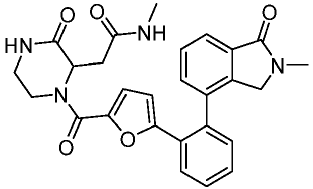 |
| Compound 36 | 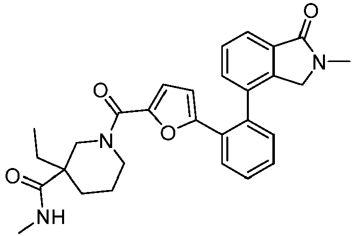 |
| Compound 37 | 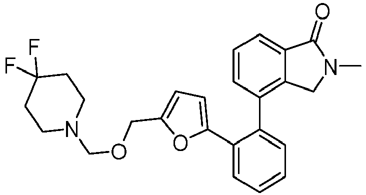 |

8. The compound of Chemical Formula II, the solvate, the stereoisomer or the pharmaceutically acceptable salt thereof of claim 5, wherein the compound is selected from the group consisting of compounds represented by the following Chemical Formulas:
| | Chemical Formula |
|---|---|
| Compound 38 | 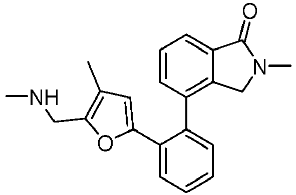 |
| Compound 39 | 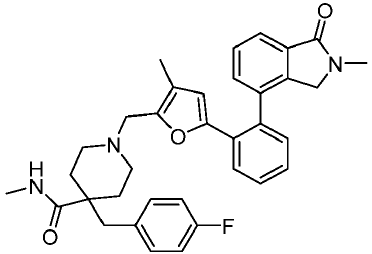 |
| Compound 40 | 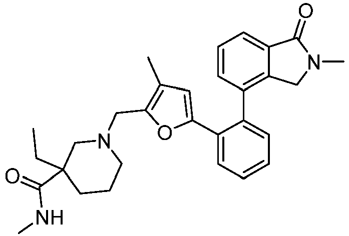 |
| Compound 41 | 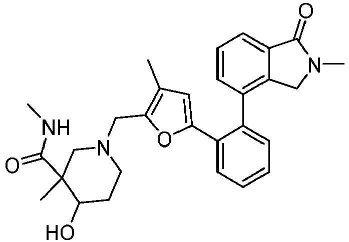 |
| Compound 42 | 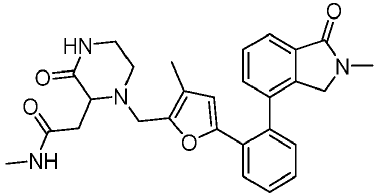 |

9. A pharmaceutical composition for preventing or treating ophthalmic diseases comprising the compound of any one of claims 1 to 8, the solvate, the stereoisomer, or the pharmaceutically acceptable salt thereof as an active ingredient.

10. The pharmaceutical composition for preventing or treating ophthalmic diseases of claim 9, wherein the ophthalmic disease is endophthalmitis, keratitis, conjunctivitis, keratoconjunctivitis, uveitis, blepharitis, scleritis, iritis, glaucoma, retinal degeneration, retinitis pigmentosa, retinal detachment, retinal pigment epithelium detachment, retinal break, diabetic retinopathy, retinopathy of prematurity, polypoidal choroidal vasculopathy, ischemic proliferative retinopathy, cone dystrophy, proliferative vitreoretinopathy, retinal artery occlusion, retinal vein occlusion, Leber's optic atrophy, corneal neovascularization, retina choroidal neovascularization, wet and dry macular degeneration, or age-related macular degeneration.

11. The pharmaceutical composition for preventing or treating ophthalmic diseases of claim 9, wherein the ophthalmic disease is diabetic retinopathy, glaucoma, uveitis, wet and dry macular degeneration, or age-related macular degeneration.
